# EUROPEAN PATENT APPLICATION

(11) **EP 3 685 838 A1**
(43) Date of publication of application: **29.07.2020**
(21) Application number: 18859489.9
(22) Date of filing: 21.09.2018
(51) Int. Cl.: A61K 31/4355, A61P 3/04, C07K 14/47

(54) **COMPOUND FOR TREATMENT OR PREVENTION OF OBESITY OR DISEASES RELATED TO OBESITY, AND APPLICATION THEREOF**

(30) Priority: 21.09.2017 CN 201710861971
(71) Applicant: Shanghai Institutes For Biological Sciences, Chinese Academy Of Sciences, Shanghai 200031 (CN); Kunming Institute Of Botany, The Chinese Academy Of Sciences, Kunming, Yunnan 650201 (CN)
(72) Inventor: ZHOU, Yunfu, Shanghai 200031 (CN); LI, Lin, Shanghai 200031 (CN); HAO, Xiaojiang, Kunming Yunnan 650201 (CN); CHEN, Duozhi, Kunming Yunnan 650201 (CN)
(74) Representative: Keenway Patentanwälte Neumann Heine Taruttis
(86) International application number: PCT/CN2018/107067
(87) International publication number: WO 2019/057175

(57) **Abstract**

A compound for treatment or prevention of obesity or diseases related to obesity, and an application thereof. Specifically, provided are a compound as represented by formula I, or a pharmaceutically-acceptable salt thereof, and a pharmaceutical composition containing the compound. The compound has multiple functions and can be used for treatment or prevention of obesity or diseases related to obesity.

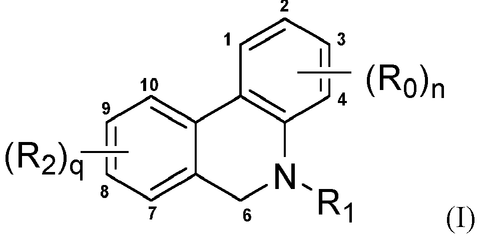

## Description

### Technical field

The present invention relates to the field of medicinal chemistry, and more particularly, to a compound for treating or preventing obesity or a related disease thereof and application thereof.

### Background

Chronic diseases such as obesity, cancer, cardiovascular disease, and neurodegenerative diseases are major health threats in modern human society, which seriously affects human health and quality of life, and at the same time, which also brings huge economic pressure to the society. Obesity also has complications such as cardiovascular disease, diabetes, and hypertension. Therefore, drug treatment to reduce weight is beneficial to obesity itself and obesity-related complications, but in the past few decades we have witnessed that too many drugs have been forced to go out of the market due to serious toxic side effects, such as causing pulmonary hypertension, cardiovascular system toxicity, neurological and mental problems. Until now, the selection of safe and effective drugs for treating obesity still faces serious challenges.

Obesity, defined excessive accumulation of individual fat, is quantified as a body mass index (BMI, equals to body weight (kg) divided by the square of height (m)) greater than 30, which is already a global biomedical problem, although it was mainly popular in North America, Europe and other Western developed countries. However, according to the statistics of the World Health Organization, about 13% of the world's population is the obesity. The number of the obesity from 85.7 million in 1980 to 210 million in 2013, the total doubled in about 20 years. Take the particularly serious United States as an example, between 2013 and 2014, about one-third of the total population was the obesity. Among them, the obesity rate of men was about 35%, and the obesity rate of women was as high as 40%. Obesity is accompanied by a series of complications, such as type 2 diabetes, cardiovascular disease and hypertension. At the same time, it has a high risk of fatty liver, kidney disease, stroke, cancer, osteoarthritis, infertility, etc., which seriously affects human health and quality of life, but also brings huge economic pressure to society. There are currently three main ways to deal with obesity: (1) lifestyle adjustments, such as reducing diet, increasing exercise, etc., (2) surgical treatments, such as changing the area of gastrointestinal absorption, thereby reducing energy input, and (3) medication. However, the history of the development of drugs for the treatment of obesity is a rather unsuccessful process. Almost all drugs for the treatment of obesity are accompanied by serious side effects, which causes most drugs to be off the market soon after they are marketed. 2,4-Dinitrophenol (DNP), first reported to have a good weight-loss effect in 1933, at the time, it was used by 100,000 people in the United States, but due to high fever, rapid heartbeat, and neural responses, death and other cases, it was stopped in 1938. Thyroid Hormone has been used to treat obesity since the 1950s. It was later discovered that the decreased part was mainly non-fatty tissue, mainly reduced water content, and had side effects on the heart, muscles and bones so that it was off market.

The discovery of *Leptin* has set off a wave of research into the field of molecular genetics for obesity and energy metabolism. Leptin is mainly synthesized and secreted by adipocyte in white adipose tissue (WAT). Leptin acts on the brain and peripheral organs or tissues. It functions to control food intake, promote energy consumption, regulate neuroendocrine and immunity and the like. Originally people hoped to use Leptin to treat obesity, but later has found that Leptin itself is not suitable for the treatment of obesity. Although the level of serum Leptin in many obese patients is quite high, it does not play a role, manifested as Leptin impedance or Leptin insensitivity. Drugs currently targeting Leptin include Leptin analogs such as MetreLeptin (trade name of MYALEPT) and sensitizers of Leptin such as triptolide and dextrin (Amylin). Leptin as a target for weight loss drug development will encounter problems such as Leptin impedance or the failure of Leptin molecules and signaling pathways, so that it is very important to find new targets.

Perhaps because nutritional intake and storage are so important for the survival of individuals, it is so difficult to develop weight loss drugs to regulate this process, which also makes the obesity drug market have a huge vacant. Although there are many side effects, obesity puts so much pressure and burden on various tissues and organs of the human body, so that for many obese people, weight loss is imperative.

Therefore, the present invention urgently needs to develop a small molecule compound for weight loss aimed at a new target.

### Summary of the invention

The object of the present invention is to provide a small molecule compound for weight loss aimed at a new target.

In a first aspect of the present invention, it provides a compound of formula I, or a salt thereof, or an optical isomer thereof, or a racemate thereof, or a solvate thereof, or a precursor thereof, 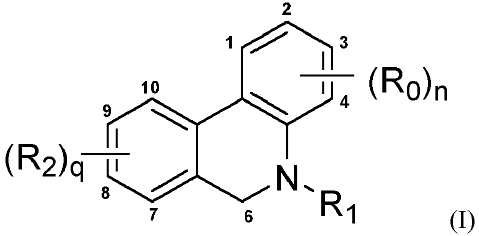 wherein
Each R₀ is independently selected from the group consisting of: H, halogen, substituted or unsubstituted C1-C8 alkyl, substituted or unsubstituted C2-C8 alkenyl, substituted or unsubstituted C2-C8 alkynyl, substituted or unsubstituted C₃-C₈ cycloalkyl, substituted or unsubstituted C1-C8 alkoxy, R3-C (O)-, R4-C (O) -O-CH₂-, R₅ (R₆) N-, substituted or unsubstituted 5-20 membered heterocyclyl, -OH, and substituted or unsubstituted benzyl; wherein the heterocyclyl contains 1-3 heteroatoms selected from N, O, and S, and the substituted means that H in the group is substituted by one or more substituents selected from the group consisting of: -OH, oxo (= O), -CN, nitro, carboxyl, halogen, C1-C3 alkyl, C1-C3 haloalkyl, and -NRcRd; n is 1, 2, 3 or 4, preferably 1, 2 or 3, more preferably 1 or 2;
R1 is selected from the group consisting of: H, substituted or unsubstituted C₁-C₃₀ alkyl, substituted or unsubstituted C₂₋C₂₀ alkenyl, substituted or unsubstituted C₂-C₂₀ alkynyl, substituted or unsubstituted -O -C₁-C₂₀ alkyl, substituted or unsubstituted C₃-C₈ cycloalkyl, -OH, substituted or unsubstituted-(CH₂)ₘ-C6-C12 aryl or C4-C10 heteroaryl and substituted or unsubstituted benzyl; the heteroaryl contains 1-3 heteroatoms selected from N, O, S, and the substituted means that the H in the group is substituted by one or more substituents selected from the group consisting of: halogen, -OH, oxo (= O), -CN, -NRcRd, and nitro;
Each R2 is independently selected from the group consisting of: -O-R₇, wherein R₇ is selected from the group consisting of: H, substituted or unsubstituted C1-C8 alkyl, substituted or unsubstituted C2-C8 alkenyl, substituted or unsubstituted C2-C8 alkynyl,-(CH₂)ₘ-substituted or unsubstituted C6-C12 aryl or C4-C10 heteroaryl and substituted or unsubstituted C₃-C₈ cycloalkyl; the heteroaryl contains 1 -3 heteroatoms selected from N, O, S, and the substituted means that H in the group is substituted by one or more substituents selected from the group consisting of: C1-C8 alkoxy, C1-C8 alkyl, halogen, -OH, oxo (= O), -CN, -NRcRd, and nitro; m is a positive integer of 0-6, preferably 1, 2, 3, or 4;
q is 0, 1, 2, 3 or 4;
R3 and R4 are each independently selected from the group consisting of: H, C1-C8 alkyl, C2-C8 alkenyl, C2-C8 alkynyl, C₃-C₈ cycloalkyl, halogen, -OH, -CN, -NRcRd, nitro, and a combination thereof;
R5 and R6 are each independently selected from the group consisting of: H, C1-C8 alkyl, C2-C8 alkenyl, C2-C8 alkynyl, C₃-C₈ cycloalkyl, -OH, -CN; or R5 and R6 together with the adjacent N form a 4-8 membered nitrogen-containing heterocyclic ring;
or each R2 together with the connected C atom may form a substituted or unsubstituted 5-20 membered heterocyclyl containing at least one oxygen atom, and the 5-20 membered heterocyclyl contains 1-3 heteroatoms selected from N, O, S, wherein the substituted means having one or more substituents selected from the group consisting of: C1-C8 alkoxy, C1-C8 alkyl, halogen, -OH, -CN, -NRcRd, and nitro;
Rc and Rd are each independently H, C₁-C₃ alkyl, C3-C6 cycloalkyl.

In another preferred embodiment, the any substituted means that H in the group is substituted by one or more substituents selected from the group consisting of -OH, oxo (= O), -CN, nitro, carboxyl, halogen, C1-C3 alkyl, C1-C3 haloalkyl, and -NRcRd; wherein, Rc and Rd are each independently H, C1-C3 alkyl, C3-C6 cycloalkyl.

In another preferred embodiment, the compound has a structure as shown in Formula Ia: 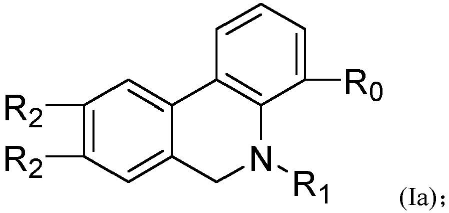 wherein the definitions of R0, R1, and each R2 are as described above.

In another preferred embodiment, the compound has a structure as shown in Formula Ib: 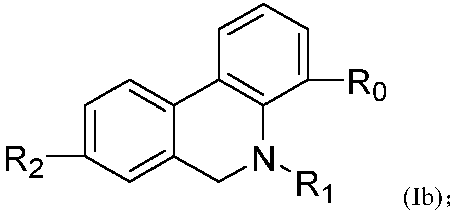 wherein, the definitions of R0, R1, and R2 are as described above.

In another preferred embodiment, the compound has a structure as shown in Formula Ic: 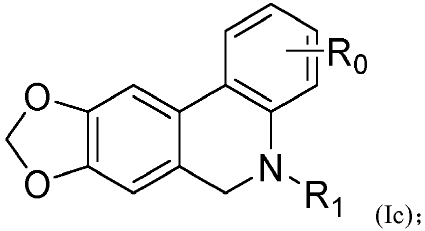 wherein the definitions of R0 and R1 are as described above.

In another preferred embodiment, the compound has a structure as shown in Formula Id: 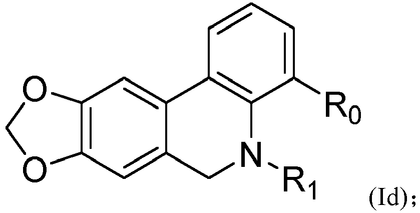 Wherein, the definitions of R0 and R1 are as described above.

In another preferred embodiment, each R₀ is independently selected from the group consisting of: H, halogen, substituted or unsubstituted C1-C6 alkyl, substituted or unsubstituted C2-C6 alkenyl, substituted or unsubstituted C2 -C6 alkynyl, substituted or unsubstituted C₃-C₆ cycloalkyl, substituted or unsubstituted C1-C6 alkoxy, R3-C (O)-, R4-C (O) -O-CH₂-, R₅ (R₆) N-, substituted or unsubstituted 5-10 membered heterocyclyl, -OH, substituted or unsubstituted benzyl, and a combination thereof; wherein, the heterocyclyl contains 1-3 heteroatoms selected from N , O, S, and the substituted means that H in the group is substituted by one or more substituents selected from the group consisting of: -OH, oxo (= O), carboxyl, halogen, C1-C3 alkyl, C1-C3 alkoxy, C1-C3 haloalkyl, and -NH₂; n is a positive integer of 1-4, preferably a positive integer of 1-3;
R1 is selected from the group consisting of: H, substituted or unsubstituted C₁-C₂₅ alkyl, substituted or unsubstituted C₂-C₁₀ alkenyl, substituted or unsubstituted C₂-C₁₀ alkynyl, substituted or unsubstituted -O -C₁-C₁₅ alkyl, substituted or unsubstituted C₃-C₆ cycloalkyl, -OH, substituted or unsubstituted-(CH₂)ₘ-C6-C12 aryl or C4-C10 heteroaryl, and a combination thereof; the heteroaryl contains 1-3 heteroatoms selected from N, O, and S, and the substituted means that H in the group is substituted by one or more substituents selected from the group consisting of: halogen, -OH , -CN, -NH₂, and nitro; m is 0-6, preferably 1-4;
Each R2 is independently selected from the group consisting of: -O-R₇, wherein R₇ is selected from the group consisting of: H, substituted or unsubstituted C1-C6 alkyl, substituted or unsubstituted C2-C6 alkenyl, substituted or unsubstituted C2-C6 alkynyl, -(CH₂)ₘ-substituted or unsubstituted C6-C12 aryl or C4-C10 heteroaryl, substituted or unsubstituted C₃-C₆ cycloalkyl, and a combination thereof; the heteroaryl contains 1-3 heteroatoms selected from N, O, S, and the substituted means that H in the group is substituted by one or more substituents selected from the group consisting of: C1-C6 alkoxy, C1-C6 alkyl, halogen, -OH, -CN, -NH₂, and nitro; m is 0-6, preferably 1-4;
R3, R4, R5 and R6 are each independently selected from the group consisting of: H, C1-C6 alkyl, C2-C6 alkenyl, C2-C6 alkynyl, C₃-C₆ cycloalkyl, halogen, -OH, -CN, -NH₂, nitro, and a combination thereof;
or each R2 together with the connected C atom may form a substituted or unsubstituted 5-10 membered heterocyclyl containing at least one oxygen atom, and the 5-10 membered heterocyclyl contains 1-3 heteroatoms selected from N, O, S, wherein the substituted means having one or more substituents selected from the group consisting of: C1-C6 alkoxy, C1-C6 alkyl, halogen, -OH, -CN, -NH₂, and nitro.

In another preferred embodiment, R₀ is 1, 2 or 3 groups or substituents independently selected from the group consisting of: H, halogen, substituted or unsubstituted C₁-C₅ alkyl, substituted or unsubstituted C₂-C₅ alkenyl, substituted or unsubstituted C₂-C₅ alkynyl, substituted or unsubstituted C₃-C₈ cycloalkyl, -OH, substituted or unsubstituted -OC₁-C₅ alkyl, R₅(R₆)N-, substituted or unsubstituted benzyl, and a combination thereof; and the substituted means that H in the group is substituted by one or more substituents selected from the group consisting of: halogen, -OH, -CN, -NH₂ , and nitro, R5 and R6 are each independently H, C₁-C₅ alkyl.

In another preferred embodiment, R₁ is selected from the group consisting of: H, substituted or unsubstituted C₁-C₅ alkyl, substituted or unsubstituted C₂-C₅ alkenyl, substituted or unsubstituted C₂-C₅ alkynyl, substituted or unsubstituted C₃-C₈ cycloalkyl, -OH, substituted or unsubstituted benzyl, and a combination thereof; and the substituted means that H in the group is substituted by one or more substituents selected from the group consisting of: halogen, -OH, -CN, -NH₂, and nitro.

In another preferred embodiment, R0 is one or two groups or substituents independently selected from the group consisting of: H, halogen, substituted or unsubstituted C₁-C₄ alkyl, substituted or unsubstituted C₂- C₄ alkenyl, substituted or unsubstituted C₂-C₄ alkynyl, substituted or unsubstituted C₃-C₆ cycloalkyl, -OH, substituted or unsubstituted -OC₁-C₄ alkyl, -NRaRb, substituted or unsubstituted benzyl, and a combination thereof; and the substituted means that H in the group is substituted by one or more substituents selected from the group consisting of: halogen, -OH, -CN, -NH₂, and nitro;
wherein, Ra and Rb are each independently H, C₁-C₃ alkyl, C3-C6 cycloalkyl, or Ra and Rb together with the adjacent N form a 4-6 membered nitrogen-containing heterocyclic ring.

In another preferred embodiment, R1 is selected from the group consisting of: H, substituted or unsubstituted C₁-C₂₀ alkyl, substituted or unsubstituted C2-C20 alkenyl, substituted or unsubstituted C2-C20 alkynyl, substituted or unsubstituted C₃-C₈ cycloalkyl, -OH, substituted or unsubstituted benzyl, and a combination thereof; and the substituted means that H in the group is substituted by one or more substituents selected from the group consisting of: halogen, -OH, -CN, -NRaRb, and nitro.

In another preferred embodiment, R₁ is selected from the group consisting of: H, substituted or unsubstituted C₁-C₃ alkyl, substituted or unsubstituted C₂-C₄ alkenyl, substituted or unsubstituted C₂-C₄ alkynyl, substituted or unsubstituted C₃-C₆ cycloalkyl, -OH, substituted or unsubstituted benzyl, and a combination thereof; and the substituted means that H in the group is substituted by one or more substituents selected from the group consisting of: halogen, -OH, -CN, -NRaRb, and nitro.

In another preferred embodiment, R₇ is selected from the group consisting of: H, substituted or unsubstituted C₁-C₂₀ alkyl, substituted or unsubstituted C₁-C₂₀ alkoxy, substituted or unsubstituted C2-C20 alkenyl, substituted or unsubstituted C2-C20 alkynyl, substituted or unsubstituted C₃-C₈ cycloalkyl, substituted or unsubstituted benzyl, and a combination thereof; and the substituted means that H in the group is substituted by one or more substituents selected from the group consisting of: halogen, -OH, oxo (= O), -CN, -NRaRb, and nitro.

In another preferred embodiment, each R0 may be the same or different.

In another preferred embodiment, the R0 is H, substituted or unsubstituted C₁-C₄ alkyl, substituted or unsubstituted C₂-C₄ alkenyl, substituted or unsubstituted C₂-C₄ alkynyl, substituted or unsubstituted C₃-C₆ cycloalkyl, R3-C(O)-, R4-C(O)-O-CH₂-, substituted or unsubstituted C₁-C₄ alkoxy, -NRaRb, substituted or unsubstituted benzyl, or

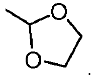

In another preferred embodiment, the R0 is one group or substituent.

In another preferred example, R0 is selected from the group consisting of: methyl, propenyl, ethyl, vinyl, and methoxy.

In another preferred embodiment, R1 is selected from the group consisting of: H, substituted or unsubstituted C₁-C₂₀ alkyl, substituted or unsubstituted C2-C20 alkenyl, substituted or unsubstituted C2-C20 alkynyl, substituted or unsubstituted C₃-C₈ cycloalkyl.

In another preferred embodiment, R1 is methyl.

In another preferred embodiment, R2 is -O-R₇, wherein R₇ is H, substituted or unsubstituted C₁-C₁₀ alkyl, substituted or unsubstituted C₁-C₂₀ alkoxy, substituted or unsubstituted C2-C20 alkenyl, substituted or unsubstituted C2-C20 alkynyl, substituted or unsubstituted C₃-C₈ cycloalkyl; -(CH₂)ₘ- substituted or unsubstituted C6-C12 aryl or C4-C10 heteroaryl.

In another preferred embodiment, there are two R2 located at the 8th and 9th positions, respectively.

In another preferred embodiment, q is 2.

In another preferred embodiment, the two R2 at the 8th and 9th positions together with the adjacent C atom form a 5-7 membered heterocyclic ring, wherein the heterocyclic ring contains 1-2 oxygen atoms.

In another preferred embodiment, R1, R0 together with the connected N and C atoms form a substituted or unsubstituted 5-8 membered heterocyclic ring, wherein the heterocyclic ring contains 1-3 N atoms and 0-2 heteroatoms selected from O and S.

In another preferred embodiment, the halogen is selected from the group consisting of: F, Cl, Br and I.

In another preferred embodiment, the halogen is Cl.

In another preferred embodiment, the compound is an optical isomer or a racemate.

In another preferred embodiment, R₀, R₁, and R₂ are specific groups corresponding to the specific compounds in the examples.

In another preferred embodiment, the compound is each specific compound prepared in the examples, and the preferred compound is selected from the group consisting of: 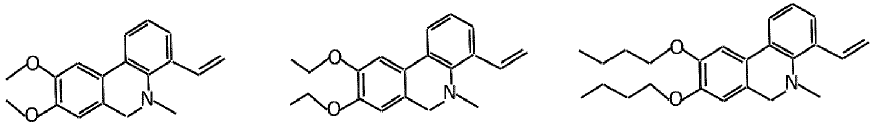 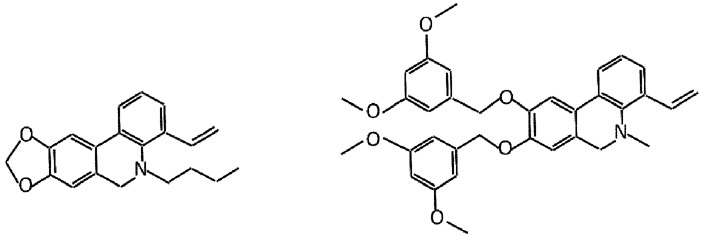 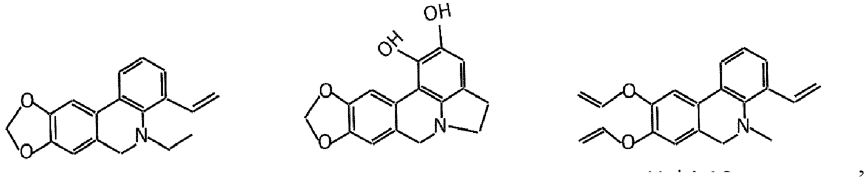 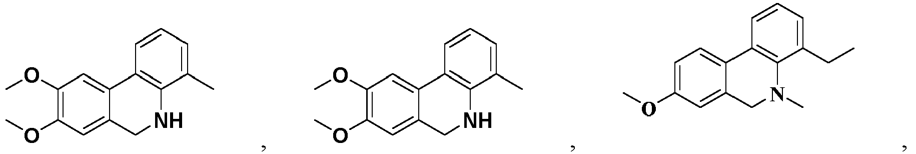 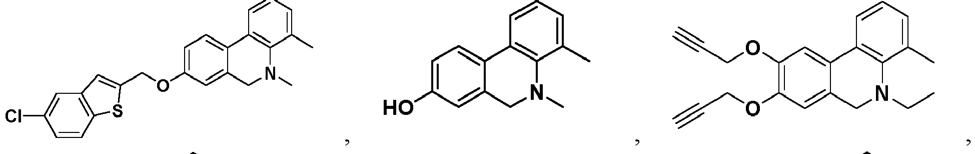 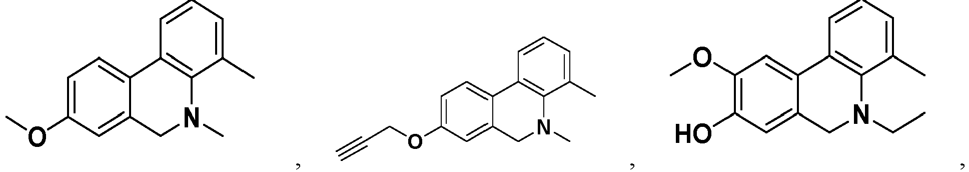 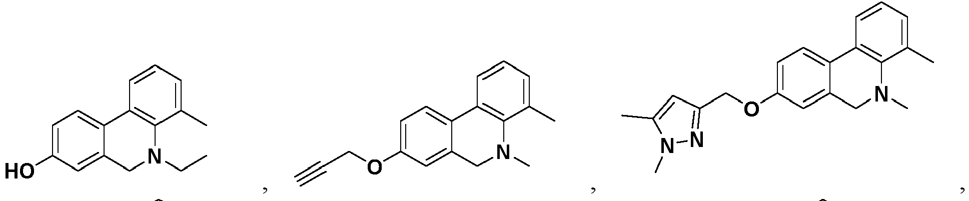 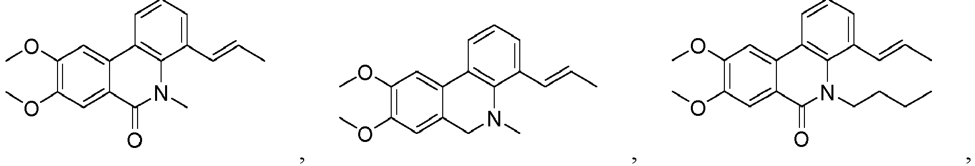 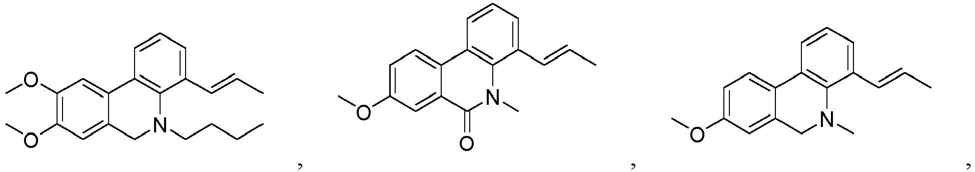 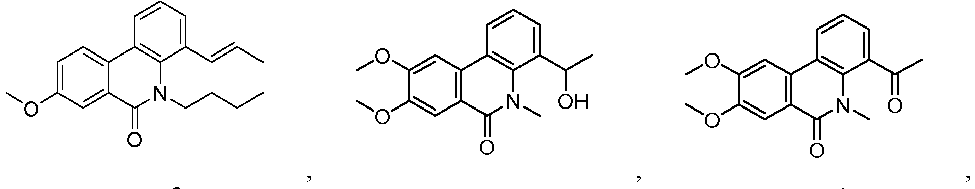 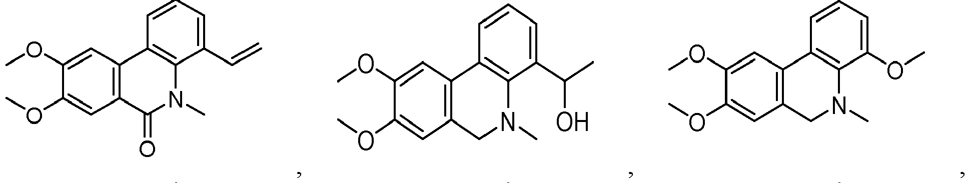 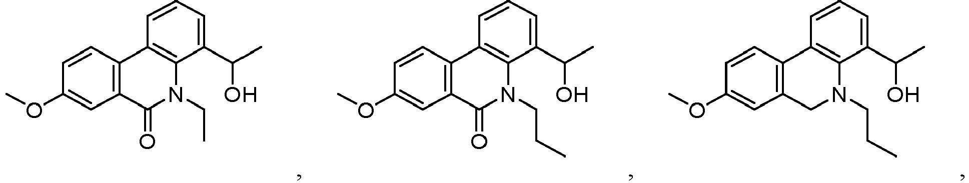 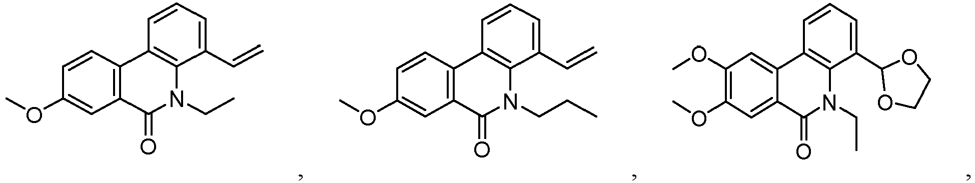 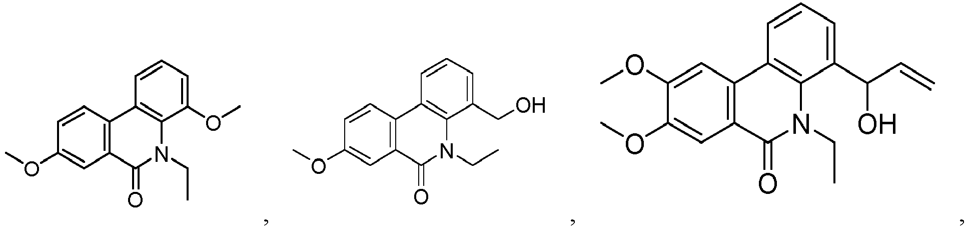 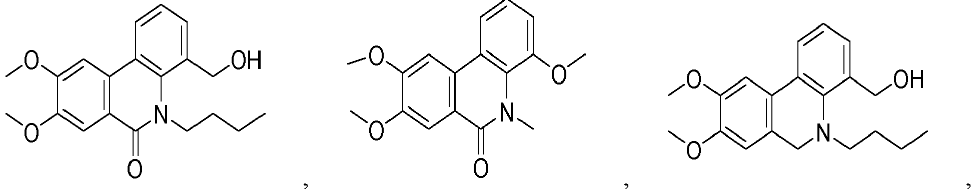 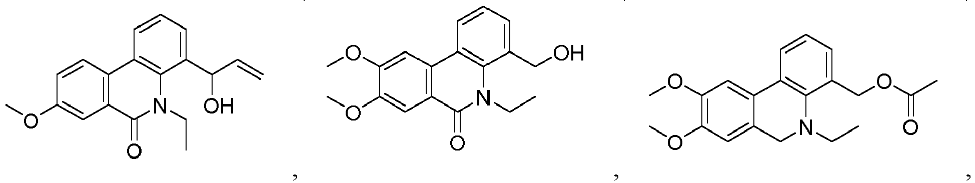 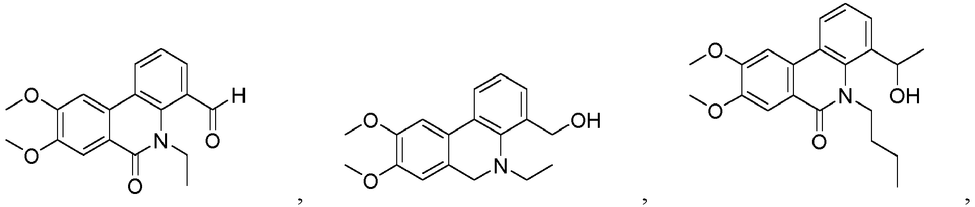 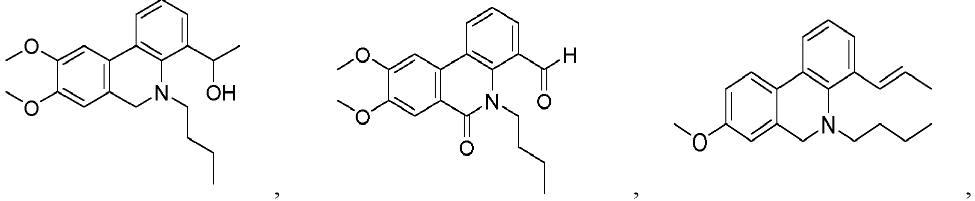 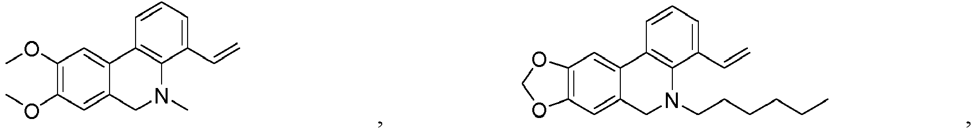 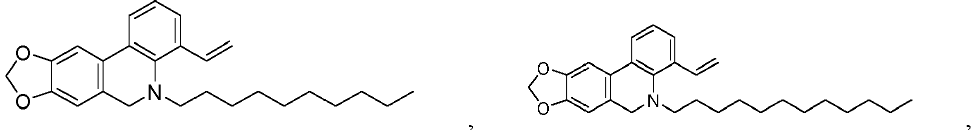 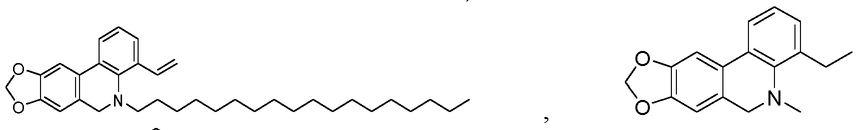 and

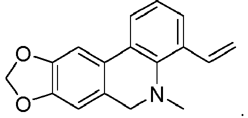

In another preferred embodiment, the compound is selected from the group consisting of:

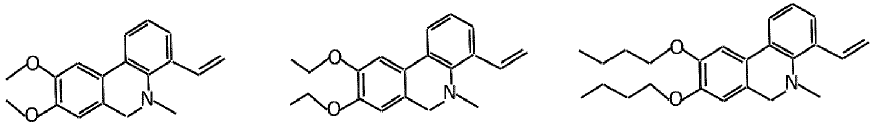

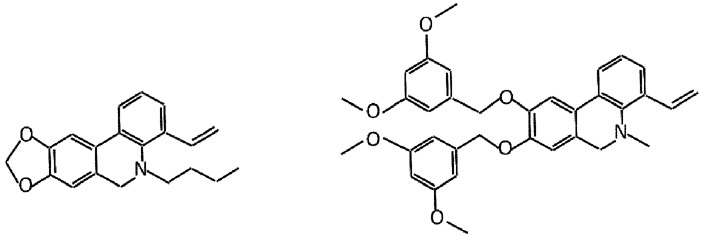

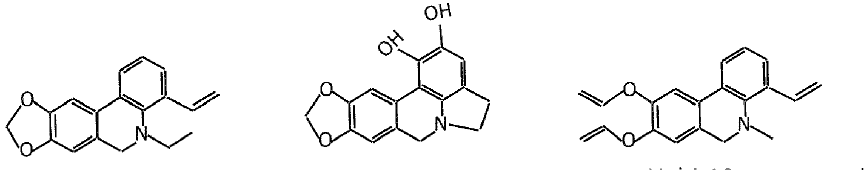

In a second aspect of the present invention, it provides a use of a compound of formula I, or a salt thereof, or an optical isomer thereof, or a racemate thereof, or a solvate thereof, or a precursor thereof for preparing a composition or preparation, the composition or preparation is used to treat or prevent obesity or a related disease thereof, wherein the compound of formula I is as described in the first aspect of the present invention.

In another preferred embodiment, the obesity or a related disease thereof is selected from the group consisting of: obesity, diabetes, hyperlipidemia, high cholesterol, fatty liver, atherosclerosis, and a combination thereof.

In another preferred embodiment, the obesity includes obesity that is not sensitive to leptin.

In another preferred embodiment, the leptin insensitivity includes Leptin impedance and / or Leptin molecule and signal pathway failure.

In another preferred embodiment, the composition or preparation is also used to (a) reduce food intake; (b) reduce body weight; (c) reduce drinking water; (d) reduce fat content; (e) improve obesity-related indicators ; (f) improve metabolism-related indicators; (g) regulate the expression of lipid metabolism genes; (h) promote the use of fat; (i) reduce fat storage and synthesis of adipose tissue; and/or (j) increase lipolysis of adipose tissue.

In another preferred embodiment, the indicators related to improving obesity include reducing one or more indicators selected from the group consisting of: blood glucose, triacylglycerol, cholesterol, alanine transaminase ALT, content of body fat, and a combination thereof.

In another preferred embodiment, the indicators related to improving metabolism includes reducing one or more indicators selected from the group consisting of: dissipation of energy, respiratory exchange rate, night physical activity, and a combination thereof.

In another preferred embodiment, the regulation of the expression of lipid metabolism genes includes reducing the expression of one or more genes selected from the group consisting of: DGAT, FAS, SCD1, and a combination thereof.

In another preferred embodiment, the regulation of the expression of lipid metabolism genes includes increasing the expression of one or more genes selected from the group consisting of: ATGL, FAT (CD36), FATP2, FATP5, CPT1A, ACADM, FATP1, FATP4, CPT1B, and a combination thereof.

In another preferred embodiment, the composition includes a pharmaceutical composition, a food composition or a health product composition.

In another preferred embodiment, the composition or preparation further includes an additional component selected from the group consisting of: other drugs for treating or preventing the obesity or a related disease thereof.

In another preferred embodiment, the other drugs for treating or preventing the obesity or a related disease thereof are selected from the group consisting of: phentermine, orlistat, Lorcaserin , Liraglutide, Topiramate, Benzphetamine, Phendimetrazine, diethylpropion, naltrexone, bupropion, and a combination thereof.

In another preferred embodiment, the pharmaceutical composition contains (i) a compound of formula I, or a salt thereof, or an optical isomer thereof, or a racemate thereof, or a solvate thereof, or a precursor thereof; and (ii) a pharmaceutically acceptable carrier.

In another preferred embodiment, the component (i) accounts for 0.001-99.9 wt% of the total weight of the pharmaceutical composition, preferably 0.1-99 wt%, more preferably 1% -90 wt%.

In another preferred embodiment, the concentration of the compound of formula I, or a salt thereof, or an optical isomer thereof, or a racemate thereof, or a solvate thereof, or a precursor thereof is 0.001ug-10000000ug / ml, preferably 0.1ug-1000000ug / ml, more preferably 10ug-100000ug / ml.

In another preferred embodiment, the composition or drug includes an oral preparation and a non-oral preparation.

In another preferred embodiment, the preparation includes: powder, granule, capsule, injection, tincture, oral liquid, tablet or lozenge.

In another preferred embodiment, the composition is an oral preparation.

In another preferred the composition (such as a pharmaceutical composition) is administered to a mammal by oral administration, intravenous injection, or local injection.

In another preferred embodiment, the mammal includes a human or non-human mammal.

In another preferred embodiment, the non-human mammal includes a rodent, such as a mouse and a rat.

In a third aspect of the present invention, it provides a pharmaceutical composition, including:
(a) The compound of formula I , or a salt thereof, or an optical isomer thereof, or a racemate thereof, or a solvate thereof, or a precursor thereof as described in the first aspect of the present invention; and
(b) a pharmaceutically acceptable carrier.

In another preferred embodiment, the pharmaceutical composition contains a therapeutically effective amount or a safe and effective amount of a compound of formula I, or a salt thereof, or an optical isomer thereof, or a racemate thereof, or a solvate thereof, or a precursor thereof.

In another preferred embodiment, the prodrug includes an ester compound.

In another preferred embodiment, the pharmaceutical composition contains 0.0001-99wt% (preferably 0.01-90wt%, more preferably 0.1-80wt%) of component (i), based on the total weight of the pharmaceutical composition.

In another preferred embodiment, the pharmaceutical composition further includes other drugs for treating or preventing the obesity or a related disease thereof.

In another preferred embodiment, the other drugs for treating or preventing the obesity or a related disease thereof are selected from the group consisting of phentermine, orlistat, Lorcaserin , Liraglutide, Topiramate, Benzphetamine, Phendimetrazine, diethylpropion, naltrexone, bupropion, and a combination thereof.

In another preferred embodiment, the pharmaceutical composition is used to prepare a drug or a preparation for treating or preventing the obesity or a related disease thereof.

In another preferred embodiment, the pharmaceutical dosage form is a dosage form for oral administration or non-oral administration.

In another preferred embodiment, the dosage form for oral administration is a tablet, pulvis, granule or capsule, or emulsion or syrup.

In another preferred embodiment, the dosage form for non-oral administration is an injection.

In a fourth aspect of the present invention, it provides a kit containing:
(i) a first container, and an active ingredient (a1) a compound of formula I, or a salt thereof, or an optical isomer thereof, or a racemate thereof, or a solvate thereof, or a precursor thereof or a drug containing the active ingredient (a1) contained in the first container; wherein the compound of formula I, or a salt thereof, or an optical isomer thereof, or a racemate thereof, or a solvate thereof, or a precursor thereof is as defined in the first aspect of the present invention.

In another preferred embodiment, the kit further includes:
(ii) a second container, and an active ingredient (a2) other drugs for treating or preventing the obesity or a related disease thereof, or a drug containing the active ingredient (a2) contained in the second container.

In another preferred embodiment, the first container and the second container are the same or different containers.

In another preferred embodiment, the drug in the first container is a prescribed preparation containing the compound of formula I, or a salt thereof, or an optical isomer thereof, or a racemate thereof, or a solvate thereof, or a precursor thereof.

In another preferred embodiment, the drug in the second container is a prescribed preparation of other drugs for treating or preventing the obesity or a related disease thereof.

In another preferred embodiment, the pharmaceutical dosage form is an oral dosage form or an injection dosage form.

In another preferred embodiment, the kit further includes instructions that describe instructions for the combined administration of the active ingredient (a1) and the active ingredient (a2) to treat or prevent the obesity or a related disease thereof.

In another preferred embodiment, the dosage form of the preparation containing the compound of formula I, or a salt thereof, or an optical isomer thereof, or a racemate thereof, or a solvate thereof, or a precursor thereof as described in the first aspect of the present invention or the preparation containing other drugs for treating or preventing the obesity or a related disease thereof includes capsules, tablets, suppositories, or intravenous injections, respectively.

In another preferred embodiment, in the preparation containing the compound of formula I or a salt thereof, or an optical isomer thereof, or a racemate thereof, or a solvate thereof, or a precursor thereof as described in the first aspect of the present invention, the concentration of the compound of formula I, or a salt thereof, or an optical isomer thereof, or a racemate thereof, or a solvate thereof, or a precursor thereof as described in the first aspect of the present invention is 0.001ug-10000000ug / ml, preferably, 0. 1ug-1000000ug / ml, more preferably, 10ug-100000ug / ml.

In a fifth aspect of the present invention, it provides an *in vitro* non-therapeutic method for (a) reducing food intake; (b) reducing body weight; (c) reducing drinking water; (d) reducing fat content; (e) improving obesity-related indicators; (f) improving indicators related to metabolism; (g) regulating the expression of lipid metabolism genes; (h) promoting the utilization of fat; (i) reducing fat storage and synthesis of adipose tissue; and / or (j) increasing lipolysis of adipose tissue comprising:
(i) administering a subject in need the compound of formula I, or a salt thereof, or an optical isomer thereof, or a racemate thereof, or a solvate thereof, or a precursor thereof according to the first aspect of the present invention, or the pharmaceutical composition according to the third aspect of the present invention, or the kit according to the fourth aspect of the present invention.

In another preferred embodiment, the administration includes oral administration.

In another preferred embodiment, the administered dose is 10-30 mg / kg body weight / day, preferably, 10 mg / kg body weight / day.

In another preferred embodiment, the administration frequency is 1-2 times / day, preferably 1 time / day.

In another preferred embodiment, the administration includes one or more cycles, each cycle being 1-7 days, preferably 2-3 days.

In another preferred embodiment, the subject includes a human or non-human mammal.

In another preferred embodiment, the non-human mammal includes a roden (such as a mouse and rat) and primate (such as a monkey).

In a sixth aspect of the present invention, it provides a method for treating or preventing the obesity or a related disease thereof, comprising the steps of:
(i) administering a subject in need the compound of formula I, or a salt thereof, or an optical isomer thereof, or a racemate thereof, or a solvate thereof or a precursor thereof according to the first aspect of the present invention, or the pharmaceutical composition according to the third aspect of the present invention, or the kit according to the fourth aspect of the present invention.

In another preferred embodiment, the administration includes oral administration.

In another preferred embodiment, the administered dose is 10-30 mg / kg body weight / day, preferably, 10 mg / kg body weight / day.

In another preferred embodiment, the administration frequency is 1-2 times / day, preferably 1 time / day.

In another preferred embodiment, the administration includes one or more cycles, each cycle being 1-7 days, preferably 2-3 days.

In another preferred embodiment, the subject includes a human or non-human mammal.

In another preferred embodiment, the non-human mammal includes a rodent (such as a mouse and rat) and primate (such as a monkey)

In a seventh aspect of the present invention, it provides a method for preparing a pharmaceutical composition for treating or preventing the obesity or a related disease thereof, comprising the steps of: mixing the compound of formula I, or a salt thereof, or an optical isomer thereof, or a racemate thereof, or a solvate thereof or a precursor thereof according to the first aspect of the present invention, with a pharmaceutically acceptable carrier, thereby forming a pharmaceutical composition.

In another preferred embodiment, the pharmaceutical composition includes other drugs for treating or preventing the obesity or a related disease thereof.

In another preferred embodiment, the other drugs for treating or preventing the obesity or a related disease thereof are selected from the group consisting of phentermine, orlistat, and Lorcaserin , Liraglutide, Topiramate, Benzphetamine, Phendimetrazine, diethylpropion, naltrexone, bupropion, and a combination thereof.

In an eighth aspect of the present invention, it provides a NUCB2 polypeptide fragment or a pharmaceutically acceptable salt thereof, the polypeptide fragment has the following characteristics:
(i) the sequence of the polypeptide fragment is derived from the amino acid sequence of the NUCB2 polypeptide; and
(ii) the polypeptide fragment is the Mth to Nth positions of the full-length amino acid sequence of the NUCB2 polypeptide, which is 40-50 amino acids in length, wherein M is any positive integer of 75-85 and N is any positive integer of 110-125.

In another preferred embodiment, the amino acid sequence of the NUCB2 polypeptide is derived from a mouse or human.

In another preferred embodiment, the polypeptide fragment further has one or more (or all) characteristics selected from the group consisting of:
(iii) the polypeptide fragment has the ability to bind the compound of formula I as described in the first aspect of the present invention;
(iv) the polypeptide fragment is an important regulatory region of NUCB2 protein function.

In another preferred embodiment, P_{M-N} represents a polypeptide composed from the Mth to Nth positions of the amino acid sequence of the NUCB2 polypeptide.

In another preferred embodiment, M is 80, 81, 82, 83, or 84.

In another preferred embodiment, M is 82 or 83.

In another preferred embodiment, N is 120, 121, 122, 123, 124, or 125.

In another preferred embodiment, N is 122.

In another preferred embodiment, the accession number of the amino acid sequence of the wild-type NUCB2 polypeptide is mouse: NP_001123951.1, and / or human: NP_005004.1.

In another preferred embodiment, the N-terminal and / or C-terminal of the polypeptide fragment also carries a tag sequence (such as 6His, or HIS6-SUMO-TEV fragment) for purification and separation purposes.

In another preferred embodiment, the polypeptide fragment is a polypeptide fragment obtained by expression using yeast as a host.

In another preferred embodiment, the polypeptide fragment is recombinant.

In another preferred embodiment, the polypeptide is expressed in E. coli BL21.

In a ninth aspect of the present invention, it provides a fusion protein comprising the NUCB2 polypeptide fragment according to the eighth aspect of the present invention and a tag sequence fused with the fragment.

In another preferred embodiment, the tag sequence is fused to the N-terminal and / or C-terminal of the polypeptide fragment.

In another preferred embodiment, the tag sequence is selected from the group consisting of His6, His6-SUMO, and His6-SUMO-TEV fragments.

In a tenth aspect of the present invention, it provides a polynucleotide sequence encoding the polypeptide fragment according to the eighth aspect of the present invention, or the fusion protein according to the ninth aspect of the present invention.

In an eleventh aspect of the present invention, it provides an expression vector containing a polynucleotide encoding the polypeptide fragment according to the eighth aspect of the present invention.

In another preferred embodiment, the vector is E. coli BL21.

In a twelfth aspect of the present invention, it provides a host cell selected from the group consisting of:
(a) a host cell containing the vector according to the eleventh aspect of the present invention;
(b) a host cell in which a polynucleotide encoding the polypeptide fragment according to the eighth aspect of the present invention is integrated into a chromosome.

In another preferred embodiment, the host cell is selected from the group consisting of a yeast cell, E. coli, an insect cell, a mammalian cell, and a combination thereof.

In a thirteenth aspect of the present invention, it provides a method for preparing the polypeptide fragment according to the eighth aspect of the present invention, comprising the steps of:
(a) cultivating the host cell according to the twelfth aspect of the present invention under conditions suitable for expression, thereby expressing the polypeptide fragment according to the eighth aspect of the present invention; and
(b) isolating the polypeptide fragment according to the eighth aspect of the present invention from the culture product.

In a fourteenth aspect of the present invention, it provides a method for screening a candidate compound for treating or preventing the obesity or a related disease thereof, comprising the steps of:
(a) Mixing the NUCB2 polypeptide fragment or a pharmaceutically acceptable salt thereof according to the eighth aspect of the present invention with a compound library, and determining the binding of the compound in the compound library to the NUCB2 polypeptide fragment or a pharmaceutically acceptable salt thereof;
   wherein, if the compound in the test compound library binds to the NUCB2 polypeptide fragment or a pharmaceutically acceptable salt thereof, indicating that the compound that binds to the NUCB2 polypeptide fragment or a pharmaceutically acceptable salt thereof is a candidate compound;
(b) administering the candidate compound determined in step (a) to a non-human animal model, and measuring the effect of the candidate compound on the obesity or a related disease thereof in a non-human animal model.

In another preferred embodiment, the step (b) further includes the step (c): comparing the candidate compound determined in step (b) with a positive control compound, the positive control compound is a compound of formula I or a pharmaceutically acceptable salt thereof, wherein the compound of formula I is as described in the first aspect of the present invention; detecting the effect of the candidate compound and the positive control compound on the amount of food intake, body weight, water consumption, fat content, obesity-related indicators, metabolism-related indicators, expression of lipid metabolism genes, fat utilization, fat storage and synthesis of adipose tissue, and / or lipolysis of adipose tissue in a non-human animal models.

In another preferred embodiment, the method is non-diagnostic and non-therapeutic.

It should be understood that, within the scope of the present invention, each technical feature of the present invention described above and in the following (as examples) may be combined with each other to form a new or preferred technical solution, which is not listed here due to space limitations.

### Description of Figure

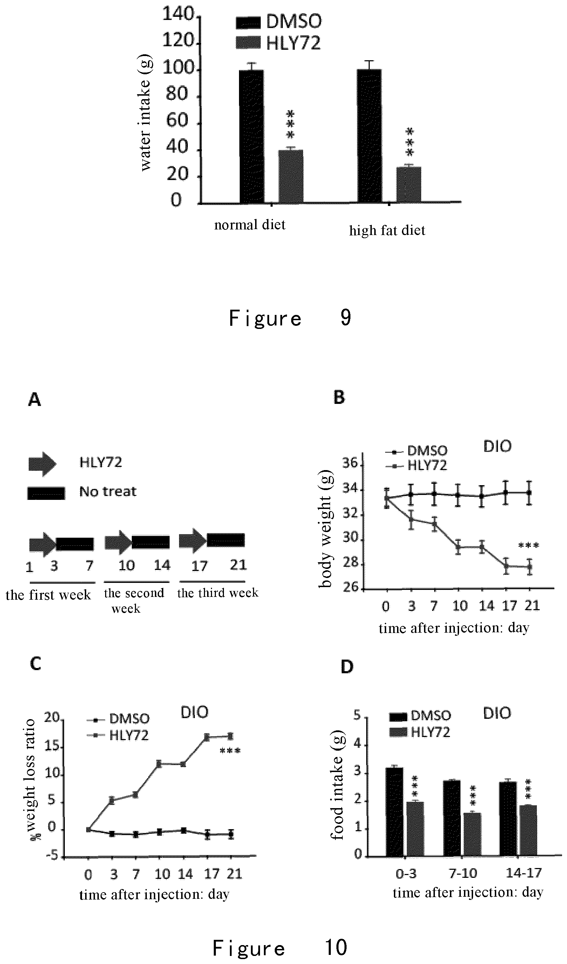

**Figure 1** **HLY78 specifically binds to NUCB2**
   Among them, (A) Bio78 binds to NUCB2-HA expressed by 293T cells (C) Ca²⁺ is important for binding of purified NUCB2 protein to Bio78 (D) purified NUCB2 protein binds to HLY78 but not to Bio-EZC.
**Figure 2** **HLY78 reduces the food intake of mice**
   Among them, (A) the food intake by mice at 1, 3, 6, 12, and 24 hours after intraperitoneal injection of 20 mg / kg HLY78 molecule (8 mice per group) (B) the body weight of the two groups of mice two days before and the day of injection of HLY78 (8 mice per group) (C, D) three days before HLY78 injection, the food intake by mice in 3 and 24 hours after injection of DMSO (8 mice per group) .

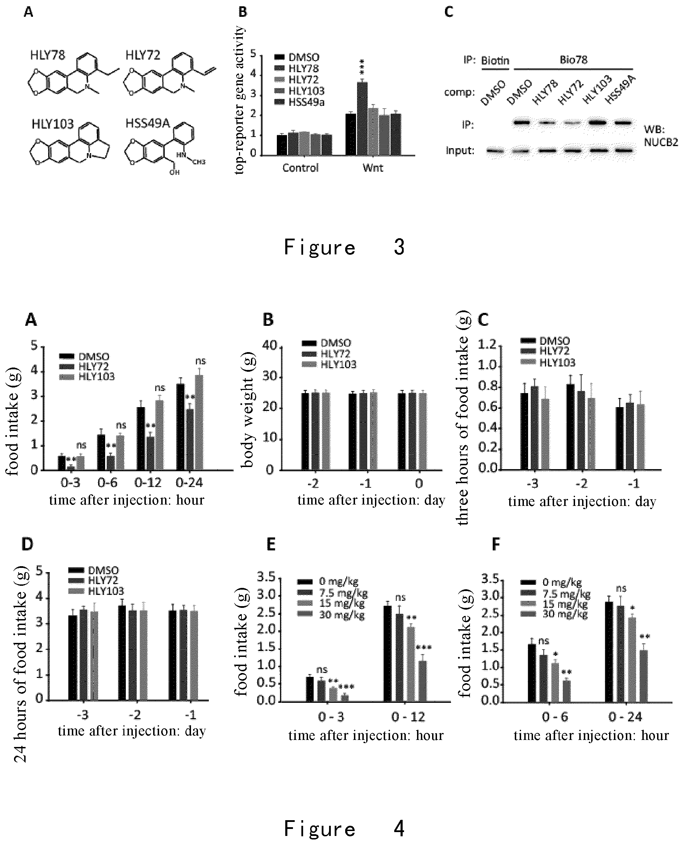

**Figure 3** **HLY78 analog HLY72 has the function of reducing eating, while HLY103 and HSS49A cannot**
   Among them, (A) molecular structure of HLY72, HLY103, HSS49A (B) HLY72, HLY103, HSS49A have no Wnt activation activity (C) HLY78, HLY72 can compete for the binding of Bio78 and NUCB2, but HLY103, HSS49A cannot.
**Figure 4** **HLY72 reduces the food intake of mice**
   Among them, (A) HLY72 reduces the food intake of mice within 1, 3, 6, 12, and 24 hours after intraperitoneal injection of 20 mg / kg while HLY103 has no effect (8 mice per group) (B) body weight of two groups of mice two days before injection and day of injection of HLY72, HLY103 (8 mice per group) (C, D) food intake of 2 groups of mice 3 days before HLY72, HLY103 injection and injection of DMSO in 3 and 24 hours (8 mice per group) (E, F) HLY72 dose-dependently reduces the food intake of mice (8 mice per group).

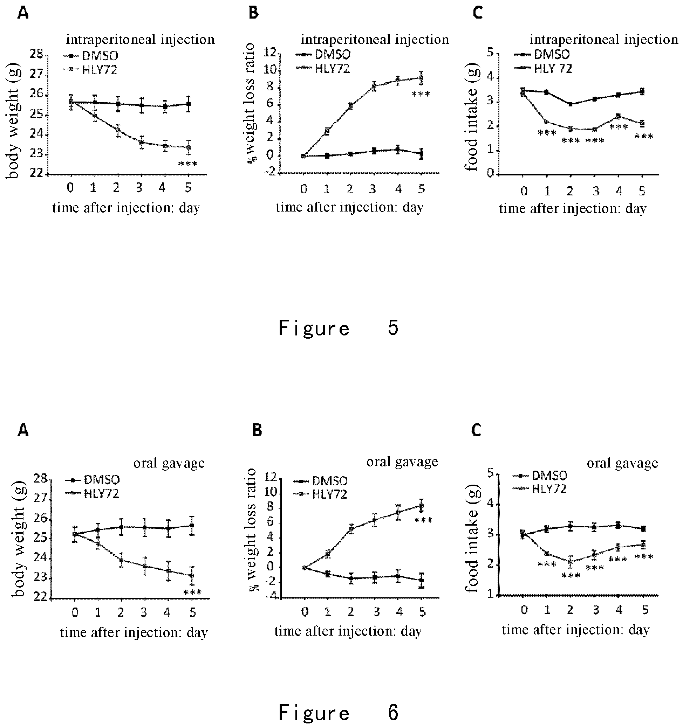

**Figure 5** **Intraperitoneal injection of HLY72 can reduce the weight of mice**
   Intraperitoneal injection (i.p.) of 20 mg / kg HLY72 for 5 consecutive days, (A) body weight of mice, (B) percentage of weight loss, (C) daily food intake (12 mice per group)
**Figure 6** **Oral gavage of HLY72 can reduce the weight of mice**
   Oral gavage of HLY72 at 20 mg / kg for 5 consecutive days, (A) body weight of mice, (B) percentage reduction in body weight, (C) daily food intake (12 mice per group).

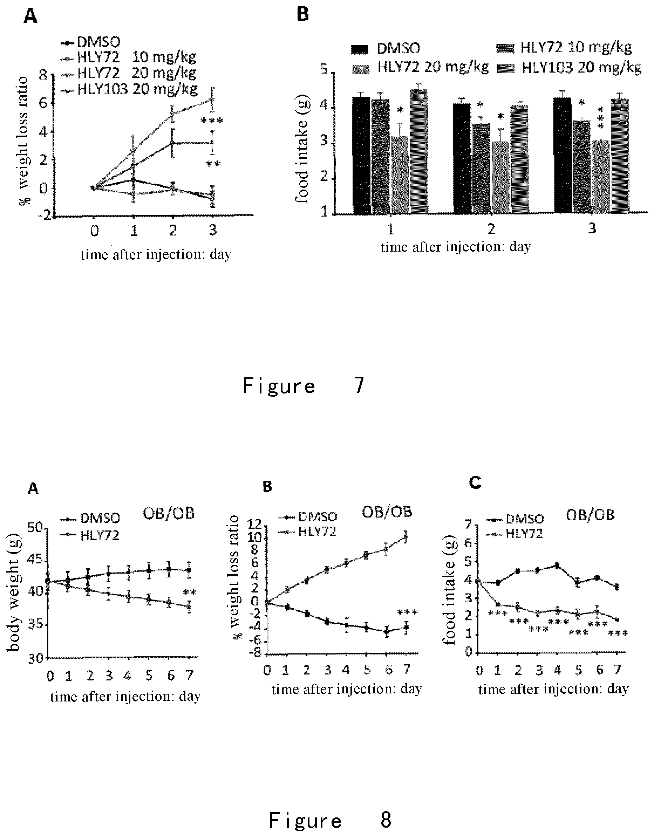

**Figure 7** **HLY72 concentration gradient dependently reduces the body weight of mice, while HLY103 cannot**
   Intraperitoneal injection of HLY72 at 10mg / kg and 20mg / kg and HLY103 at 20mg/kg for 3 consecutive days (A) 3 days of mice body weight (B) 3 days of mice food intake (6 mice per group).
**Figure 8** **The effect of HLY72 on weight loss in OB / OB obese model mice**
   (A) body weight, (B) percentage of weight loss, (C) daily food intake of mice (8 mice in DMSO group, 10 mice in HLY72 group) of OB / OB obese mice treated with 20 mg / kg HLY72
**Figure 9** **HLY72 reduces water consumption in mice**
**Figure 10** **Effect of HLY72 on weight loss in obese mice induced by high fat**
   (A) Pattern of periodic intermittent administration, (B, C, D) weight, weight loss percentage, daily food intake of high-fat-induced obese mice under the treatment of 20 mg / kg HLY72 (9 mice per group).

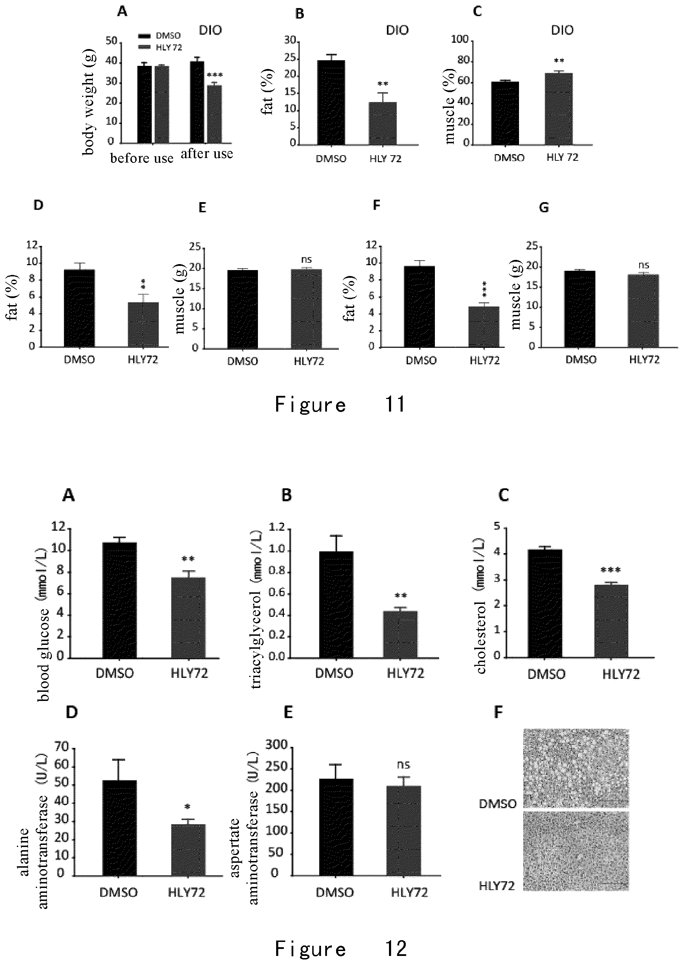

**Figure 11** **HLY72 significantly reduces the fat content of obese mice and normal mice**
   (A) Before and after using 3 courses of 20 mg / kg HLY72, body weight of two groups of mice (6 mice in DMSO group, 6 mice in HLY72 group) (B, C) After HLY72 is used, the fat content and muscle content of mice are changed (4 mice in the DMSO group and 6 mice in the HLY72 group) (D, E) Normal mice are intraperitoneally injected with 20 mg / kg of HLY72. After 5 days, the mice fat content and muscle content are changed (8 mice in the DMSO group , 8 mice in the HLY72 group) (F, G) Normal mice are orally administrated with 20 mg / kg of HLY72. After 5 days, the fat content and muscle content of the mice are changed (12 mice in the DMSO group and 12 mice in the HLY72 group) .
**Figure 12** **HLY72 improves obesity and accompanied diseases**
   After using 20 mg / kg HLY72, (A) blood glucose, (B) blood triacylglycerol, (C) cholesterol, (D) alanine transaminase, (E) glutamic oxalacetic transaminase (4 mice in DMSO group, 6 mice in HLY72 group) are changed (F) Hematoxylin and eosin staining of liver slices (scale, 200µm) in DMSO group and HLY72 group.

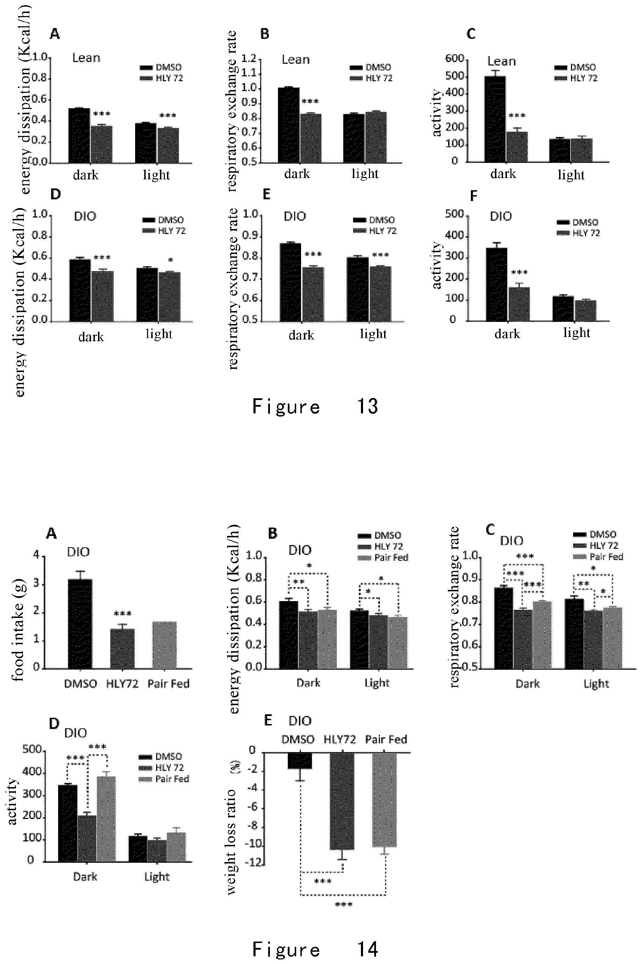

**Figure 13** **Effect of HLY72 on metabolism**
   Mice fed with normal diet (A, B, C) (14 mice per group), mice fed with high-fat diet (D, E, F) (14 mice per group) are placed in the metabolic cage for 3 days, the energy dissipation (A, D), respiratory exchange rate (B, E), and physical activity (C, F) of HLY72 or DMSO mice injected with 20 mg / kg once a day.
**Figure 14** **Paired feeding experiment**
   Mice fed with high-fat diet (8 mice per group) are placed in a metabolic cage for 3 days and injected with 20 mg / kg of HLY72 or DMSO once a day. Half of the mice in the DMSO group are paired with the HLY72 group. The average daily food intake (A), energy dissipation (B), respiratory exchange rate (C), physical activity (D) (E) of the three groups of mice after the three-day metabolic cage experiment is completed, the weight of the three groups of mice.

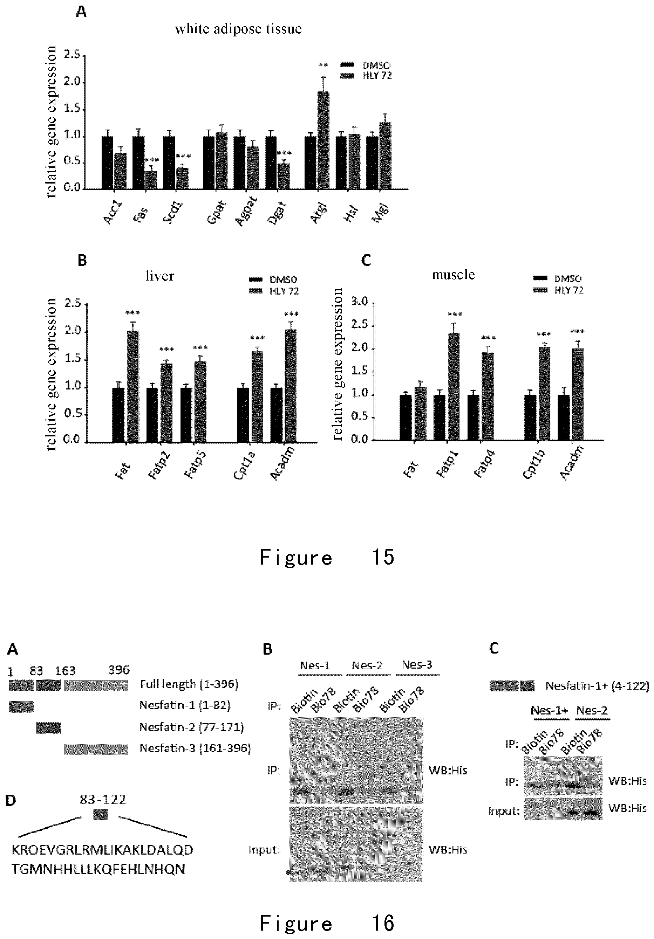

**Figure 15** **Effect of HLY72 on gene expression related to lipid metabolism**
   Mice fed with normal diet are injected with 20 mg / kg of HLY72 for 3 consecutive days, and the white adipose tissue (A) is taken to detect fat storage, fatty acid synthesis and decomposition-related gene expression. Liver (B) is taken to detect fatty acid transport and oxidative gene expression. Muscle (C) is taken to test the fatty acid transport and oxidative gene expression (14 mice per group).
**Figure 16** **Amino acids 83-122 of NUCB2 are the key sites for HLY72 binding**
   (A) Schematic diagram of NUCB2 protein fragmentation (B) The main binding fragment of Bio78 is Nesfatin-2 (C) Nesfatin-1 + has a stronger ability to bind Bio78 than Nesfatin-2 (D) The amino acid sequence from position 83 to position 122 of the NUCB2 protein.

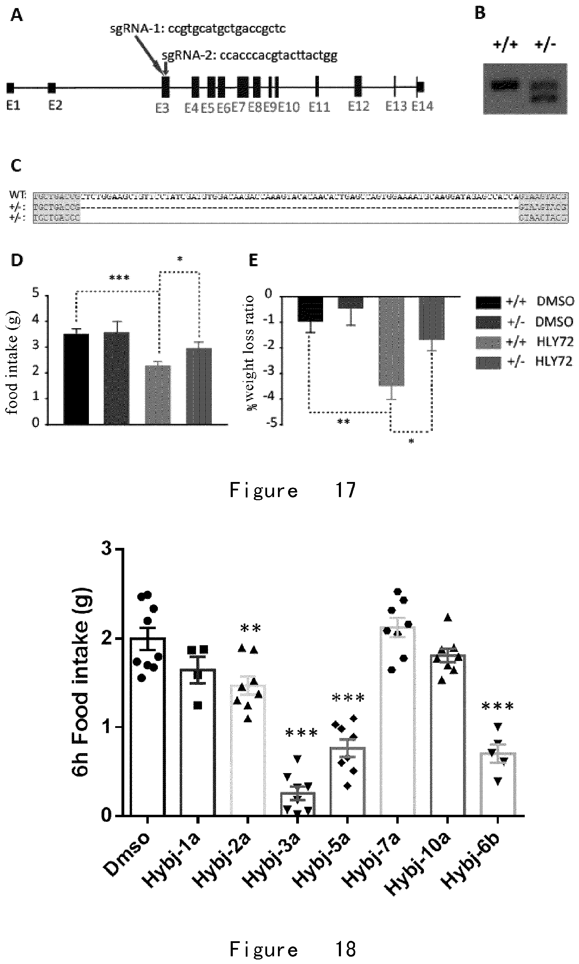

**Figure 17** **The effect of HLY72 on NUCB2 gene knockout heterozygous mice is reduced**
   (A) Mouse NUCB2 gene information and sgRNA sequence (B) PCR identification of heterozygous NUCB2 knockout mouse lines (C) sequencing identification of heterozygous mouse lines (D) HLY72 weakens the effect of reducing food intake in NUCB2 heterozygous mice (E) The effect of HLY72 on body weight reduction of NUCB2 heterozygous mice is weakened.
Figure 18 shows the effect of some HLY72 derivatives of the present invention on the food intake reduction of mice. All compounds are injected intraperitoneally with a concentration of 75umol / kg (20 mg / kg of Hly72 equals 75 umol / kg). One-way ANOVA statistical analysis, *: P <0.05, **: P • <0.01, *** : P <0.001.

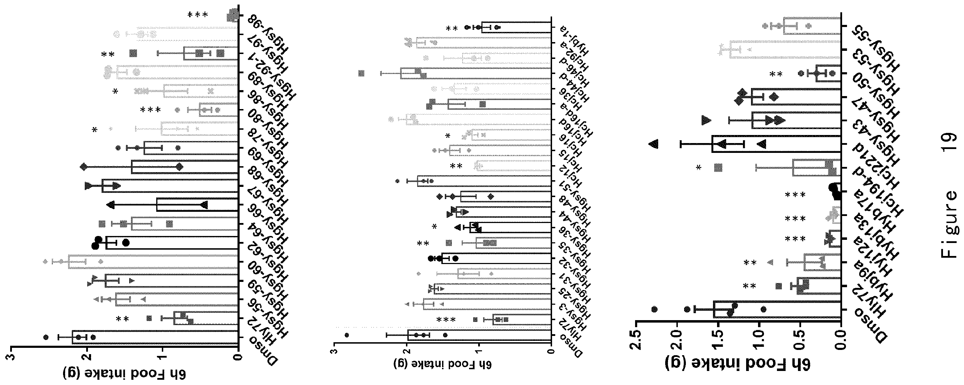

Figure 19 shows the effect of other HLY72 derivatives of the present invention on the food intake reduction of mice. All compounds are injected intraperitoneally at a concentration of 75umol / kg (20mg / kg of Hly72 equals 75umol / kg). One-way ANOVA statistics Analysis, *: P <0.05, **: P • <0.01, ***: P <0.001.

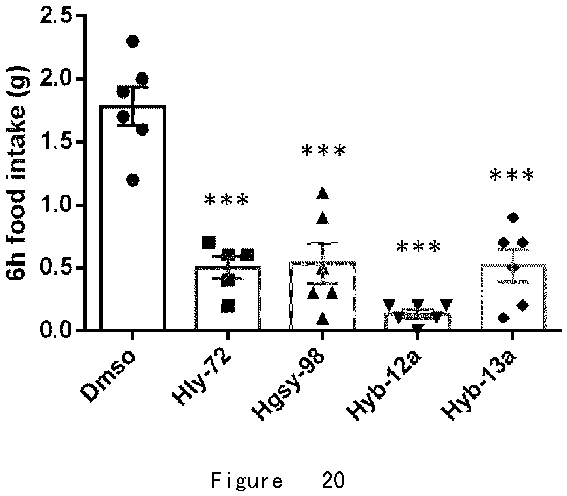

Figure 20 shows activity comparison of the compounds of the present invention Hly72 and Hgsy-98, Hybj-12a, Hybj-13a. Intraperitoneal injection of Hly72 at a concentration of 75 umol / kg (20 mg / kg of Hly72 equals 75 umol / kg), Hgsy-98 , Hybj-12a, Hybj-13a are all 7.5 umol / kg. One-way ANOVA statistical analysis, *: P <0.05, **: P • <0.01, ***: P <0.001.

### Detailed Description

After extensive and in-depth research, the inventors have unexpectedly screened for the first time a class of compounds that can specifically target NUCB2 protein to treat obesity or a related disease thereof through extensive screening. Experiments show that the compound of formula I, or a pharmaceutically acceptable salt thereof of the present invention, has the effect of (a) reducing food intake; (b) reducing body weight; (c) reducing drinking water; (d) reducing fat content; (e) improving obesity-related indicators; (f) improving metabolism-related indicators; (g) regulating the expression of lipid metabolism genes; (h) promoting the utilization of fat; (i) reducing fat storage and synthesis in adipose tissue; and / or (j) increasing lipolysis of adipose tissue. In addition, the compounds of the present invention also have excellent therapeutic effects on leptin-insensitive obesity.

In addition, the applicant has discovered for the first time that the amino acids 82-122 or 83-122 of the NUCB2 polypeptide are the key binding sites of the compound of formula I of the present invention. The compound of formula I of the present invention binds to the key sites of NUCB2 polypeptide, thereby more effectively treating or preventing the obesity or a related disease thereof. On this basis, the inventors have completed the present invention.

### Group definition

As used herein, the term "substituted or unsubstituted" means that the group may be unsubstituted, or the H in the group is substituted by one or more (such as 1-10, preferably 1-5 , more preferably 1-3, most preferably 1-2) substituents.

As used herein, "substitution" or "substituted" means that the group has one or more (preferably 1-6, more preferably 1-3) substituents selected from the group consisting of: hlogen, C₁-C₆ alkyl, C₁-C₆ haloalkyl, C₂-C₆ alkenyl, C₂-C₆ alkynyl.

As used herein, the term "C₁-C₂₀ alkyl" refers to a linear or branched alkyl group having 1-8 carbon atoms, such as methyl, ethyl, propyl, isopropyl, butyl, isobutyl, Sec-butyl, tert-butyl, or the like.

As used herein, the term "C₁-C₈ alkyl" refers to a linear or branched alkyl group having 1-8 carbon atoms, such as methyl, ethyl, propyl, isopropyl, butyl, isobutyl, sec-butyl, tert-butyl, or the like.

As used herein, the term "C₁-C₅ alkyl" refers to a linear or branched alkyl group having 1 to 5 carbon atoms, such as methyl, ethyl, propyl, isopropyl, butyl, isobutyl, sec-butyl, tert-butyl, or the like.

As used herein, the term "C₁-C₄ alkyl" refers to a linear or branched alkyl group having 1 to 4 carbon atoms, such as methyl, ethyl, propyl, isopropyl, butyl, isobutyl, sec-butyl, tert-butyl, or the like.

As used herein, the term "C₁-C₃ alkyl" refers to a linear or branched alkyl group having 1 to 3 carbon atoms, such as methyl, ethyl, propyl, isopropyl, or the like.

As used herein, the term "C₂-C₂₀ alkenyl" refers to a linear or branched alkenyl group having 2 to 20 carbon atoms, such as ethenyl, allyl, 1-propenyl, isopropenyl, 1-butenyl, 2-butenyl, or the like.

As used herein, the term "C₂-C₈ alkenyl" refers to a linear or branched alkenyl group having 2-8 carbon atoms, such as ethenyl, allyl, 1-propenyl, isopropenyl, 1-butenyl, 2-butenyl, or the like.

As used herein, the term "C₂-C₅ alkenyl" refers to a linear or branched alkenyl group having 2-5 carbon atoms, such as ethenyl, allyl, 1-propenyl, isopropenyl, 1-butenyl, 2-butenyl, or the like.

As used herein, the term "C₂-C₄ alkenyl" refers to a linear or branched alkenyl group having 2-4 carbon atoms, such as ethenyl, allyl, 1-propenyl, isopropenyl, 1-butenyl, 2-butenyl, or the like.

As used herein, the term "C₂-C₂₀ alkynyl" refers to a linear or branched alkynyl group having 2 to 20 carbon atoms, such as ethynyl, propynyl, or the like.

As used herein, the term "C₂-C₈ alkynyl" refers to a linear or branched alkynyl group having 2 to 8 carbon atoms, such as ethynyl, propynyl, or the like.

As used herein, the term "C₂-C₅ alkynyl" refers to a linear or branched alkynyl group having 2 to 5 carbon atoms, such as ethynyl, propynyl, or the like.

As used herein, the term "C₂-C₄ alkynyl" refers to a linear or branched alkynyl group having 2 to 4 carbon atoms, such as ethynyl, propynyl, or the like.

As used herein, the term "C₁-C₂₀ alkoxy" refers to a group having a (C₁-C₂₀ alkyl) -O- structure, for example, CH₃-O-, C₂H₅-O-, C₃H₈-O-, or the like.

As used herein, the term "C₁-C₈ alkoxy" refers to a group having a (C₁-C₈ alkyl) -O-structure, for example, CH₃-O-, C₂H₅-O-, C₃H₈-O-, or the like.

As used herein, the term "C₁-C₅ alkoxy" refers to a group having a (C₁-C₅ alkyl) -O-structure, for example, CH₃-O-, C₂H₅-O-, C₃H₈-O-, or the like.

As used herein, the term "C₃-C₈ cycloalkyl" refers to a cyclic alkyl group having 3-8 carbon atoms, such as cyclopropyl, cyclobutyl, cyclopentyl, cyclohexyl, cycloheptyl, or the like.

As used herein, the term "C₃-C₆ cycloalkyl" refers to a cyclic alkyl group having 3 to 6 carbon atoms, such as cyclopropyl, cyclobutyl, cyclopentyl, cyclohexyl, cycloheptyl, or the like.

As used herein, the term "C₆-C₁₂ aryl" refers to a monovalent aromatic carbocyclic group of 6 to 12 carbon atoms, which has a single ring (such as phenyl) or a fused ring (such as naphthyl or anthryl), if the connection point is on the aromatic carbon, and the fused ring may be non-aromatic (such as 2-benzoxazolone, 2H-1,4-benzoxazine-3 (4H) -one-7-yl, etc.). The substituted or unsubstituted C₅-C₂₀ aryl group is selected from the group consisting of ortho-substituted phenyl, meta-substituted phenyl, and para-substituted phenyl. Preferred aryl groups include phenyl and naphthyl. The term includes substituted or unsubstituted forms, wherein the substituents are as defined above. The substituent of the substituted phenyl is selected from the group consisting of halogen, hydroxy, methyl, ethyl, isopropyl, t-butyl, methoxy, ethoxy, t-butoxy, trifluoromethyl, and a combination thereof.

As used herein, the term "C₄-C₁₀ heteroaryl" refers to an aromatic group having 4 to 10 carbon atoms and 1 to 4 heteroatoms selected from oxygen, nitrogen, and sulfur, such heteroaryl groups may be monocyclic (such as pyridyl or furyl) or fused ring (such as indolizinyl or benzothienyl), wherein the fused ring may be non-aromatic and / or contain a hetero atom as long as the point of attachment is through an aromatic heteroaryl atom. Preferred heteroaryl groups include pyridyl, pyrrolyl, indolyl, thienyl and furyl. The term includes substituted or unsubstituted heteroaryl.

As used herein, the term "5-20 membered heterocyclyl ", or "C₅-C₂₀ heterocyclyl" refers to a saturated, partially saturated or unsaturated group (but not aromatic), having a single ring or a fused ring (including bridge ring system and spiro ring system, having 5 to 20 carbon atoms (preferably 5 to 10 carbon atoms) and 1 to 4 heteroatoms selected from nitrogen, sulfur, or oxygen, in fused ring system, one or more rings may be cycloalkyl, aryl or heteroaryl as long as the point of attachment passes through a non-aromatic ring. The term includes substituted or unsubstituted heterocyclyl.

As used herein, the term "halogen" refers to fluorine, chlorine, bromine, or iodine, preferably fluorine and chlorine.

The term "halogenated" refers to a group substituted by the same or different one or more of the above halogen atoms, which may be partially halogenated or fully halogenated, such as trifluoromethyl, pentafluoroethyl, heptafluoroisopropyl, or the like.

As used herein, the term "C₁-C₃ haloalkyl" refers to a linear or branched alkyl group having 1-3 carbon atoms in which hydrogen is substituted by one or more halogens, for example, halomethyl, haloethyl, halopropyl, halogenated isopropyl, or the like, preferably trifluoromethyl.

The compounds of the present invention may contain one or more asymmetric centers and therefore appear in the form of racemates, racemic mixtures, single enantiomers, diastereoisomeric compounds, and single diastereomers. The asymmetric centers that can exist depend on the nature of the various substituents on the molecule. Each such asymmetric center will independently produce two optical isomers, and all possible optical isomers and diastereomer mixtures and pure or partially pure compounds are included within the scope of the present invention. The present invention includes all isomeric forms of the compounds.

As used herein, the term "room temperature (r.t.)" refers to 4-40 °C, preferably 20-25 °C.

### Compounds of the present invention

As used herein, the term "compound of the present invention" refers to a compound of formula I, an optical isomer thereof, a hydrate thereof, a solvate thereof, a prodrug thereof or a pharmaceutically acceptable salt thereof.

Typically, the present invention provides a compound of formula I, an optical isomer thereof, a hydrate thereof, a solvate thereof, a prodrug thereof or a pharmaceutically acceptable salt thereof. 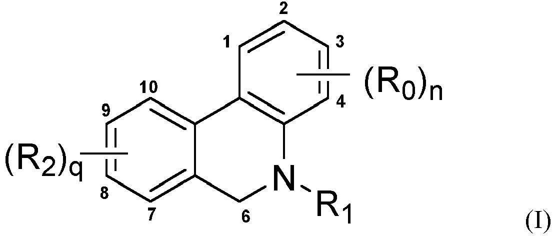 wherein
The definitions of R0, R1, R2, n, and q are as described above.

In another preferred embodiment, the compound has a structure as shown in Formula Ia: 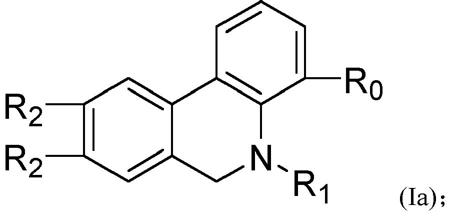 wherein, the definitions of R0, R1, and R2 are as described above.

In another preferred embodiment, the compound has a structure as shown in Formula Ib: 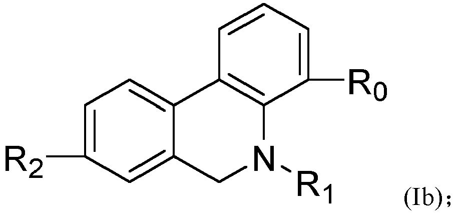 wherein, the definitions of R0, R1, and R2 are as described above.

In another preferred embodiment, the compound has a structure as shown in Formula Ic: 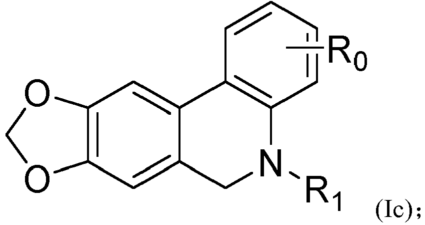 Wherein, the definitions of R0 and R1 are as described above.

In another preferred embodiment, the compound has a structure as shown in Formula Id: 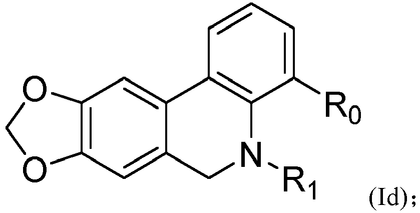 Wherein, the definitions of R0 and R1 are as described above.

In the case where the compounds as described in the present invention have stereoisomers, the present invention includes all stereoisomers of the compounds.

In the case where the compounds as described in the present invention have tautomers, the present invention includes all tautomers of the compounds.

The present invention also includes deuterated compounds produced by the replacement of any one or more hydrogen atoms in the compounds by its stable isotope deuterium.

The compounds of formula I of the present invention also include pharmaceutically acceptable salts, optical isomers, or their racemates, or their solvates, or their prodrugs (precursors).

As used herein, the term "pharmaceutically acceptable salts" refer to non-toxic acid or alkaline-earth metal salts of compounds of Formula I. These salts can be prepared in situ when the compound of Formula I is finally isolated and purified, or by reacting suitable organic or inorganic acids or bases with basic or acidic functional groups, respectively. Representative salts include, but are not limited to: acetate, adipate, alginate, citrate, aspartate, benzoate, benzenesulfonate, disulfate, butyrate, camphorate, camphorsulfonate, digluconate, cyclopentanepropionate, dodecyl sulfate, ethanesulfonate, glucose enanthate, glycerol phosphate, hemisulfate, enanthate, hexanoate, fumarate, hydrochloride, hydrobromide, hydroiodide, 2-hydroxyethanesulfonate, lactate, maleate, mesylate, nicotinate, 2-naphthylsulfonate, oxalate, pamoate, pectate, thiocyanate, 3-phenylpropionate, picrate, pivalate, propionate, succinate, sulfate, tartrate, thiocyanate, p-toluenesulfonate and undecanoate. In addition, nitrogen-containing basic groups can be quaternized with the following reagents: alkyl halides, such as a chloride, bromide, and iodide of methyl, ethyl, propyl, butyl; dialkyl sulfate, such as dimethyl, diethyl, dibutyl and dipentyl sulfate; long-chain halide such as a chloride, bromide, and iodide of decyl, lauryl, myristyl and stearyl; aralkyl halide such as bromide of benzyl and phenethyl etc.. This results in water-soluble or oil-soluble or dispersible product. Examples of acids that can be used to form pharmaceutically acceptable acid addition salts include inorganic acids such as hydrochloric acid, sulfuric acid, and phosphoric acid, and organic acids such as oxalic acid, maleic acid, methanesulfonic acid, succinic acid, and citric acid. Base addition salt can be prepared in situ when the compound of formula I is finally isolated and purified, or by reacting the carboxylic acid moiety with a suitable base (such as a pharmaceutically acceptable metal cation hydroxide, carbonate or bicarbonate) or ammonia, or an organic primary, secondary or tertiary amine. Pharmaceutically acceptable salts include, but are not limited to, alkali metal and alkaline earth metal based cations such as sodium, lithium, potassium, calcium, magnesium, aluminum salts, etc., as well as non-toxic ammonium, quaternary ammonium and amine cations, including, but not limited to: ammonium, tetramethylammonium, tetraethylammonium, methylamine, dimethylamine, trimethylamine, triethylamine, ethylamine, etc. Other representative organic amines for forming base addition salts include diethylamine, ethylenediamine, ethanolamine, diethanolamine, piperazine, and the like.

As used herein, the term "a pharmaceutically acceptable prodrug" refers to those prodrugs of the compounds of the preferred embodiments that are rapidly converted in vivo to the parent compound represented by the above formula, for example, hydrolyzed in blood. In "T. Higuchi and V. Stella, Pro-drugs as Novel Delivery Systems", Volume 14 of A.C.S. 15 Symposium Series "and "Edited by Edward B. Roche, Bioreversible Carriers in Drug Design, American Pharmaceutical Association and Pergamon Press, 1987" , provide a complete discussion, both of which are incorporated herein by reference. Preferred prodrugs are prodrugs in ester form.

### Preparation method of the compound of the present invention

The present invention provides a method for preparing the compound of formula I. The compound of the present invention can be easily prepared by various synthetic operations, which are well-known to those skilled in the art. Exemplary methods for preparing these compounds may include, but are not limited to, the schemes as described below.

Generally, in the preparation method of the present invention, each reaction is mostly performed at -20 °C to 160 °C(or reflux temperature) (preferably, -5 °C to 100 °C or 0-80 °C) for a period of time (such as 0.1-72 hours, preferably 0.5-24 hours).

Preferably, the compound of formula (I) of the present invention can be completed by the exemplary methods as described in the following schemes and examples and related published literature operations used by those skilled in the art.

In the specific operation process, the steps in the method can be expanded or merged as needed.

A typical manufacturing method is as shown in scheme I. The reaction scheme is:

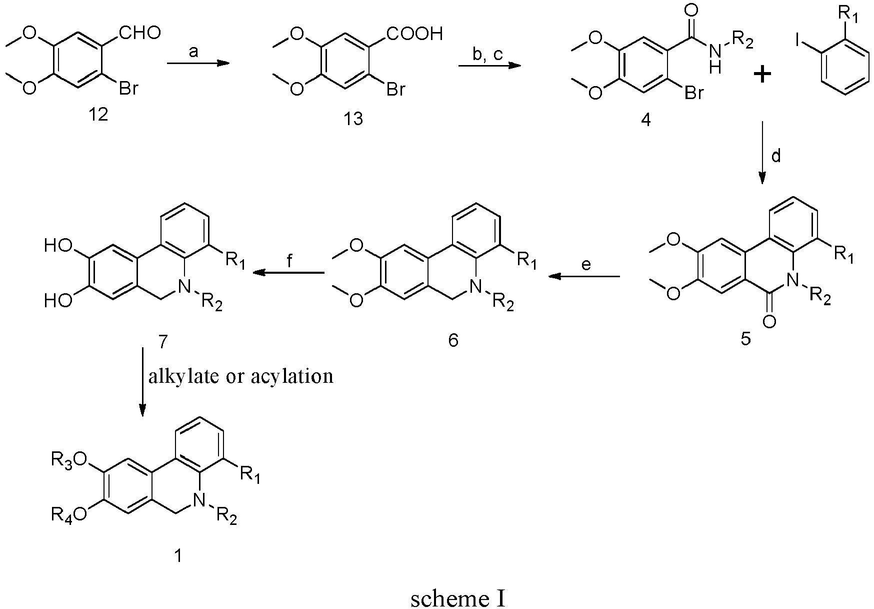

In this scheme, 2-bromo-4,5-dimethoxybenzaldehyde and 2-ethyliodobenzene or 2-vinyliodobenzene are used as raw materials through oxidation, coupling, BH₃ reduction, and demethylation steps synthesis.

Reaction reagents and conditions: a, NaHCO₃, KMnO₄, H₂O, 90 °C, 3 h, 85%; b, SOCl₂, DMF, THF, 50 °C, 2h: c, R₂NH₂ (30%), 5 °C, 1h, 75 %; d, K₂CO₃, norbornene, Pd (OAc)₂, TFP, MeCN, 85 °C, 6h, 75%; e, BH₃, THF, -78 °C, 2 h, 60%; f. BBr₃, CH₂Cl₂, -78 °C, 4h, 80%.

Another representative preparation method includes: using lycorine hydrochloride as the raw material, through N-methylation, Huffman degradation, catalytic hydrogenation and deoxymethylene reaction, as well as the preparation of alkylation and acylation of phenolic hydroxyl. This manufacturing method can be represented by scheme II:

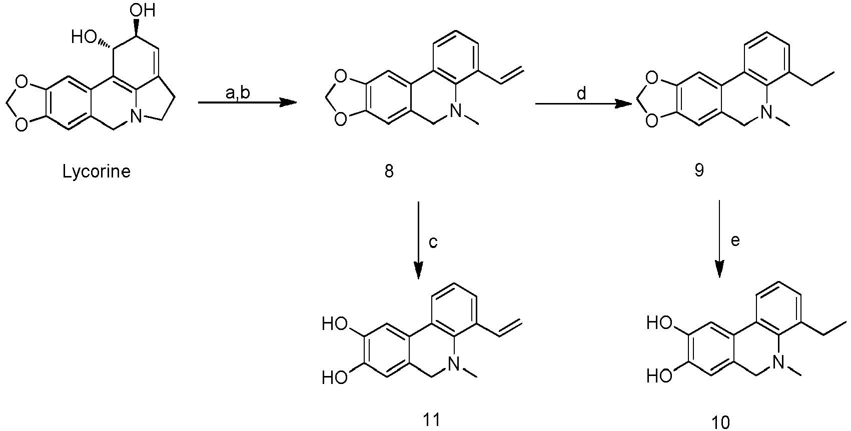

In the method for synthesizing the compound of formula (1) from lycorine, representative reaction reagents and conditions include: a, CH₃I, r.t., 12h; b, KTB, TBA, 90 °C, 4h, 90%; c, BBr₃, CH₂Cl₂, -78 °C, 6h, 65%; d, 10% Pb / C, H₂, 24h, 95%; e, BBr₃, CH₂Cl₂, -78 °C, 10h, 72%.

### NUCB2

NUCB2 (Nucleobindin2) protein NUCB2 is a secreted protein containing 420 amino acids, consisting of 24 amino acids at the amino terminus as the signal peptide, and 396 amino acids behind.

In the present invention, through extensive screening, it is found for the first time that the compound of formula I or a pharmaceutically acceptable salt thereof can effectively treat the obesity or a related disease thereof against the NUCB2 target. The compound of formula I or a pharmaceutically acceptable salt thereof has a very good druggability (an excellent solubility, bioavailability, etc.).

### NUCB2 polypeptide fragment

As used herein, the terms "polypeptide fragment of the present invention", "polypeptide fragment of NUCB2 of the present invention", "fragment of the present invention" or " short peptide of the present invention" can be used interchangeably and refers to the NUCB2 polypeptide fragment or a pharmaceutically acceptable salt thereof as defined in the present invention. In addition, it should be understood that the NUCB2 polypeptide fragments of the present invention may be derived from humans, but also from other mammals (such as rodents, such as mice or other non-human primates).

The present invention mainly provides a class of NUCB2 polypeptide fragments, which have the following characteristics:
(i) The sequence of the polypeptide fragment is derived from the amino acid sequence of the NUCB2 polypeptide (e.g., from a human or mouse); and
(ii) The polypeptide fragment is from the Mth position to the Nth position of the full-length amino acid sequence (396 amino acids) of the NUCB2 polypeptide and is 40-50 amino acids in length, wherein M is any positive integer of 75-85, and N is any positive integer from 110 to 125.

Representative polypeptide fragments of the present invention include, but are not limited to, P₈₂₋₁₂₂, P₈₃₋₁₂₂, or a combination thereof. The numbers represent the starting and ending amino acid positions in the amino acid sequence of the NUCB2 polypeptide fragment.

As used herein, "isolated" refers to the separation of a substance from its original environment (if it is a natural substance, the original environment is the natural environment). For example, polynucleotides and polypeptides in the natural state in living cells are not isolated and purified, however, if the same polynucleotide or polypeptide is separated from other substances in its natural state, it is isolated and purified.

As used herein, "isolated polypeptide fragment of the present invention" means that the polypeptide fragment of the present invention is substantially free of other proteins, lipids, carbohydrates or other substances that are naturally associated with it. Those skilled in the art can purify the polypeptide fragments of the present invention using standard protein purification techniques. A substantially pure polypeptide can produce a single main band on a non-reducing polyacrylamide gel. The purity of the polypeptide fragments of the present invention can be analyzed by amino acid sequence.

The polypeptide fragments of the present invention may be recombinant polypeptides, natural polypeptides, synthetic polypeptides, preferably recombinant polypeptides. The polypeptide fragments of the present invention may be naturally purified products including those hydrolyzed by proteases or chemically synthesized products, or using recombinant technology to produce from prokaryotic or eukaryotic hosts (e.g., bacteria, yeast, higher plants, insects, and mammalian cells). Depending on the host used in the recombinant production protocol, the polypeptide of the present invention may be glycosylated or may be non-glycosylated. The polypeptide of the present invention may further or may not include the starting methionine residue.

The present invention also includes fragments, derivatives and analogs of the polypeptide fragments of the present invention. As used herein, the terms "fragment", "derivative" and "analog" refer to a polypeptide that substantially retains the same biological function or activity as the polypeptide fragment of the present invention. The polypeptide fragment, derivative or analog of the present invention may be (i) a polypeptide having one or more conservative or non-conservative amino acid residues (preferably conservative amino acid residues) substituted, and such substituted amino acid residues may or may not be encoded by the genetic code, or (ii) a polypeptide having substitution groups in one or more amino acid residues, or (iii) a polypeptide formed by the fusion of a mature polypeptide with another compound (such as a compound that extends the half-life of the polypeptide, such as polyethylene glycol), or (iv) a polypeptide formed by fusion of additional amino acid sequence to this polypeptide sequence (such as a leader sequence or secretion sequence or the sequence used to purify this polypeptide or proprotein sequence, or fusion protein with the antigen IgG fragments). According to the teachings herein, these fragments, derivatives and analogs are within the scope of those skilled in the art.

In the present invention, the term "short peptide of the present invention" refers to a short peptide having M-N amino acids (wherein the length is 40-50 amino acids, of which M is any positive integer of 75-85, N is any positive integer of 110-125) of accession number of NP_001123951.1 or NP_005004.1 polypeptide with the polypeptide fragment activity of the present invention. The term also includes variant forms having the same function as the polypeptide fragments of the present invention. These variants include (but are not limited to): deletions, insertions and / or substitutions of one or more (usually 1-50, preferably 1-30, more preferably 1-20, most preferably 1-10) amino acids, and addition of one or several (usually within 20, preferably within 10, more preferably within 5) amino acids at the C-terminus and / or N-terminal. For example, in the art, the substitution of amino acids with close or similar properties usually does not change the function of the protein. As another example, adding one or several amino acids to the C-terminus and / or N-terminus usually does not change the function of the protein. The term also includes active fragments and active derivatives of the polypeptide fragments of the present invention.

The present invention also provides analogues of the polypeptide fragments of the present invention. The difference between these analogues and the natural short peptide of the present invention may be a difference in amino acid sequence, may also be a difference in the modification form that does not affect the sequence, or both. These polypeptides include natural or induced genetic variants. Induced variants can be obtained by various techniques, such as random mutagenesis by radiation or exposure to mutagen, or by site-directed mutagenesis or other known molecular biology techniques. Analogs also include analogs with residues different from natural L-amino acids (such as D-amino acids), as well as analogs with non-naturally occurring or synthetic amino acids (such as β, γ-amino acids). It should be understood that the polypeptide of the present invention is not limited to the representative polypeptides exemplified above.

Modified (usually without changing the primary structure) forms include: chemically derived forms of polypeptide in vivo or in vitro such as acetylation or carboxylation. Modifications also include glycosylation, such as those polypeptides produced by glycosylation modification during the synthesis and processing of the polypeptide or during further processing steps. This modification can be accomplished by exposing the polypeptide to an enzyme that performs glycosylation (such as mammalian glycosylation or deglycosylase). Modified forms also include sequences with phosphorylated amino acid residues (e.g., phosphotyrosine, phosphoserine, phosphothreonine). Also included are peptides that have been modified to improve their proteolytic resistance or optimize their solubility.

In the present invention, "conservative variant polypeptide fragment of the present invention" refers to compared with the amino acid sequence of the short peptide with M-N amino acids (wherein the length is 40-50 amino acids, of which M is any positive integer of 75-85, N is any positive integer of 110-125) of the accession number of NP_001123951.1 or NP_005004.1 polypeptide with the polypeptide fragment activity of the present invention, at most 8, preferably at most 5, more preferably at most 3, and most preferably at most 2 amino acids are replaced by amino acids with close or similar properties to form a polypeptide. These conservative variant polypeptides are preferably produced by amino acid substitution according to Table 1.

**Table 1**

| initial residue | representative substitution | preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The polynucleotide of the present invention may be in the form of DNA or RNA. DNA forms include cDNA, genomic DNA, or synthetic DNA. DNA can be single-stranded or double-stranded. DNA can be a coding strand or a non-coding strand. The coding region sequence encoding the mature polypeptide may be the same as the coding region sequence as shown in the nucleotide sequence of the NUCB2 polypeptide or a degenerate variant. As used herein, "degenerate variant" in the present invention refers to a nucleic acid sequence that encodes a polypeptide fragment having M-N amino acids of accession number of NP_001123951.1 or NP_005004.1 polypeptide with the polypeptide fragment activity of the present invention, but that differs from the coding region sequence.

Polynucleotide encoding a polypeptide having M-N amino acids of accession number of NP_001123951.1 or NP_005004.1 polypeptide with the polypeptide fragment activity of the present invention includes: a coding sequence that only encodes the mature polypeptide; a coding sequence of the mature polypeptide and various additional coding sequences; the coding sequence (and optionally additional coding sequence) and non-coding sequence of the mature polypeptide.

The polypeptide and polynucleotide in the present invention are preferably provided in isolated forms, and are more preferably purified to homogeneity.

The full-length nucleotide sequence of the short peptide of the present invention or its fragment can usually be obtained by PCR amplification method, recombination method or artificial synthesis method. For the PCR amplification method, primers can be designed according to the relevant nucleotide sequence disclosed in the present invention, especially the open reading frame sequence, and a commercially available cDNA library or a cDNA library prepared according to a conventional method known to those skilled in the art is used as a template to amplify and obtain the relevant sequences.

Once the relevant sequence is obtained, the relevant sequence can be obtained in large quantities by the recombination method. This is usually done by cloning it into a vector, then transferring it into a cell, and then isolating the relevant sequence from the propagated host cell by conventional methods.

In addition, synthetic methods can also be used to synthesize the relevant sequences, especially when the length of the fragments is short. Generally, a long sequence can be obtained by synthesizing multiple small fragments and then connecting them.

At present, the DNA sequence encoding the protein (or fragment or derivative thereof) of the present invention can be obtained completely by chemical synthesis. This DNA sequence can then be introduced into various existing DNA molecules (or such as vectors) and cells known in the art. In addition, mutations can also be introduced into the protein sequence of the present invention by chemical synthesis.

Through conventional recombinant DNA technologies (Science, 1984; 224: 1431), the polynucleotide sequences of the present invention can be used to express or produce recombinant polypeptide fragments of the present invention. Generally speaking, there are the following steps:
(1). Transforming or transducing a suitable host cell with the polynucleotide (or variant) encoding the short peptide of the present invention of the present invention, or with a recombinant expression vector containing the polynucleotide;
(2). Culturing host cell in a suitable medium;
(3). Isolating and purifying proteins from culture medium or cell.

In the present invention, the polynucleotide sequence encoding the polypeptide of the present invention can be inserted into a recombinant expression vector. The term "recombinant expression vector" refers to a bacterial plasmid, bacteriophage, yeast plasmid, plant cell virus, mammalian cell virus such as adenovirus, retrovirus, or other vectors well known in the art. Suitable vectors in the present invention include but are not limited to: T7-based expression vectors expressed in bacteria (Rosenberg, et al. Gene, 1987, 56: 125); pMSXND expression vectors expressed in mammalian cells (Lee and Nathans, J Bio Chem. 263: 3521, 1988) and vectors derived from baculovirus expressed in insect cells. In short, as long as it can replicate and stabilize in the host, any plasmid and vector can be used. An important feature of an expression vector is that it usually contains an origin of replication, a promoter, a marker gene, and a translation control element.

Vectors containing the appropriate DNA sequences and appropriate promoters or control sequences as described above can be used to transform appropriate host cells so that they can express proteins.

The host cell may be a prokaryotic cell, such as a bacterial cell; or a lower eukaryotic cell, such as a yeast cell; or a higher eukaryotic cell, such as a mammalian cell. Representative examples are: E. coli, Streptomyces; bacterial cells of Salmonella typhimurium; fungal cells such as yeast; plant cells; insect cells of Drosophila S2 or Sf9; CHO, COS, 293 cells, or animal cells of Bowes melanoma cells, etc. The preferred host cell is a yeast cell.

Transformation of host cells with recombinant DNA can be performed using conventional techniques well known to those skilled in the art. When the host is a prokaryotic organism such as E. coli, competent cells that can absorb DNA can be harvested after the exponential growth phase and treated with the CaCl₂ method. The procedures used are well known in the art. Another method is to use MgCl₂. If necessary, transformation can also be carried out by electroporation. When the host is a eukaryote, the following DNA transfection methods can be used: calcium phosphate co-precipitation method, conventional mechanical methods such as microinjection, electroporation, liposome packaging, etc.

The obtained transformant can be cultured by a conventional method and express the polypeptide encoded by the gene of the present invention. Depending on the host cell used, the medium used in the culture can be selected from various conventional mediums. The cultivation is carried out under conditions suitable for the growth of host cells. When the host cell grows to an appropriate cell density, the selected promoter is induced by an appropriate method (such as temperature conversion or chemical induction), and the cell is cultured for a period of time.

The recombinant polypeptide in the above methods may be expressed in a cell, on a cell membrane, or secreted out of the cell. If necessary, the recombinant protein can be isolated and purified by various separation methods using its physical, chemical and other characteristics. These methods are well known to those skilled in the art. Examples of these methods include, but are not limited to: conventional renaturation treatment, treatment with protein precipitation agent (salting out method), centrifugation, osmotic disruption of bacteri through osmosis, ultra-treatment, ultra-centrifugation, molecular sieve chromatography (gel filtration), adsorption chromatography, ion exchange chromatography, high performance liquid chromatography (HPLC) and various other liquid chromatography techniques and combinations of these methods.

### Nesfatin-1

In 2006, Oh-I et al. isolated a new anti-feeding neuropeptide-Nesfatin-1, from rat cerebrospinal fluid. Nesfatin-1 is actually a fragment of 1-82 amino acids at the N-terminus of NUCB2, but whether nesfatin-1 really exists is unclear. Injecting Nesfatin-1 in the third ventricle of the hypothalamus or through the peripheral intraperitoneal can inhibit the feeding of rats or mice. As one of the appetite regulating peptides, the level of serum Nesfatin-1 / NUCB2 is positively correlated with the energy level of the body. Serum Nesfatin-1 content in mouse decreases under starvation condition, and serum Nesfatin-1 content returns to normal level after refeeding. The content of Nesfatin-1 in human serum shows a positive correlation with BMI. Long-term feeding of high-fat feed to mice can increase the expression of Nesfatin-1 in adipose tissue, and stimulation of inflammatory factors such as TNFα and IL-6 can also induce the expression of Nesfatin-1 to increase.

The hypothalamic arcuate nucleus is an important area for regulating energy metabolism, which is outside the blood-brain barrier. Neurons in the arcuate nucleus can be directly stimulated by adipokines (such as Leptin, Insulin, CCK, Adiponectin, PYY, etc.) in the blood circulation, and are considered to be the first-order neurons in the regulation of feeding.

Nesfatin-1-positive neurons also exist in some areas of the brainstem. These areas include nucleus raphes pallidus and nucleus raphe obscurus, dorsal nucleus of vagus nerve, and nucleus of solitary tract. Sympathetic neuron axon originate from raphe nuclei and terminate in brown adipose tissue, implying that Nesfatin-1 plays a role in the process of sympathetic innervation of brown adipose tissue to generate heat.

The present invention identifies for the first time the small molecule compound HLY78 targeting NUCB2, and proves that HLY78 cooperates with NUCB2 to suppress appetite in a Leptin-independent manner. Long-term administration of HLY78 has a good weight loss effect on *db*/*db* mice and DIO mice.

### Composition and method of administration

The present invention also provides a pharmaceutical composition, which contains an active ingredient in a safe and effective amount range, and a pharmaceutically acceptable carrier.

The "active ingredient" in the present invention refers to the compound of the Formula I as described in the present invention, its optical isomer, hydrate, solvate, its prodrug or a pharmaceutically acceptable salt thereof.

The "active ingredient" and the pharmaceutical composition as described in the present invention can be used as agents for treating or preventing obesity or a related disease thereof. In another preferred embodiment, it is used to prepare a medicament for treating or preventing obesity or a related disease thereof. In another preferred embodiment, the obesity or a related disease thereof is selected from the group consisting of obesity, diabetes, hyperlipidemia, high cholesterol, fatty liver, atherosclerosis, and a combination thereof.

"Safe and effective amount" means that the amount of the active ingredient is sufficient to significantly improve the condition without causing serious side effects. Generally, the pharmaceutical composition contains 1-2000 mg active ingredient / dose, more preferably 10-200 mg active ingredient / dose. Preferably, the "one dose" is a tablet.

"Pharmaceutically acceptable carrier" refers to one or more compatible solid or liquid fillers or gel substances, which are suitable for human use and must have sufficient purity and sufficiently low toxicity. "Compatibility" herein means that the components of the composition can be blended with the active ingredient of the present invention and between them without significantly reducing the efficacy of the active ingredient.

The compound of the preferred embodiment of the present invention can be administered as a single active agent, or it can be used in combination with one or more other agents for treating neurodegenerative diseases. It is also effective to use the compound of the preferred embodiment of the present invention in combination with known therapeutic agents. The combination of the currently known compound and other therapeutic agents for treating neurodegenerative diseases is within the scope of the preferred embodiment. Based on the special properties of the drugs and the diseases involved, those of ordinary skill in the art can identify effective combinations of agents. Such therapeutic agents for treating neurodegenerative diseases include (but are not limited to) the following: Alzheimer's disease, Parkinson's disease, Huntington's disease, amyotrophic lateral sclerosis and the like. The compound of the preferred embodiment is also effective when administered simultaneously with a therapeutic agent for treating neurodegenerative diseases.

Generally, the compounds of the preferred embodiments will be administered in a therapeutically effective amount by any acceptable mode of agents with similar effects. The actual amount of the compound (i.e., active ingredient) of the preferred embodiment is determined according to a number of factors, such as the severity of the disease to be treated, the age and relative health of the patient, the efficacy of the compound used, the route and form of administration, and other factors. The drug can be administered multiple times a day, preferably, once or twice a day. All these factors are considered by the attending doctor.

For the purpose of the preferred embodiment, the therapeutically effective dose may generally be a total daily dose administered to the patient in one time or in divided doses, for example, about 0.001 to about 1000 mg / kg body weight per day, preferably, about 1.0 to about 30 mg / kg body weight. A unit dose composition (Dosage unit composition) may contain its dose factor to form a daily dose. The choice of dosage form depends on various factors, such as the mode of administration and the bioavailability of the drug substance. Generally, the compounds of the preferred embodiments can be administered as a pharmaceutical composition by any of the following routes: oral, systemic administration (such as transdermal, intranasal or by suppository), or parenteral administration (such as intramuscular, intravenous or subcutaneously). The preferred mode of administration is oral, and a convenient daily dose can be adjusted according to the degree of bitterness. The composition may take the form of tablets, pills, capsules, semi-solids, powders, sustained release preparation, solutions, suspensions, elixirs, aerosols, or any other suitable composition. Another preferred way to administer the compounds of the preferred embodiments is by inhalation. This is an effective method of delivering therapeutic agents directly to the respiratory tract (see, e.g., US Patent No. 5,607,915).

Suitable pharmaceutically acceptable carriers or excipients include: such as treatment agents and drug delivery modifiers and accelerators, such as calcium phosphate, magnesium stearate, talc, monosaccharides, disaccharides, starch, gelatin, cellulose , sodium methyl cellulose, carboxymethyl cellulose, glucose, hydroxypropyl-B-cyclodextrin, polyvinylpyrrolidone, low melting wax, ion exchange resin, etc., and any combination of two or more thereof. The liquid and semi-solid excipients can be selected from glycerin, propylene glycol, water, ethanol, and various oils, including petroleum, animal oil, vegetable oil, or synthetic sources, such as peanut oil, soybean oil, mineral oil, sesame oil, and the like. Preferred liquid carriers, especially carriers for injectable solutions, include water, saline, aqueous glucose solutions, and ethylene glycol. Other suitable pharmaceutically acceptable excipients are described in "Remington's Pharmaceutical Sciences", Mack Pub. Co., New Jersey (1991) and are incorporated by reference.

As used herein, the term "composition" includes (a) a composition for treating or preventing obesity or a related disease thereof, and optionally (b) other drugs for treating or preventing obesity or a related disease thereof (phentermine, orlistat, Lorcaserin, Liraglutide, Topiramate, Benzphetamine, Phendimetrazine, diethylpropion, naltrexone or bupropion); In addition, the composition includes a pharmaceutical composition, a food composition or a health product composition.

In a preferred embodiment, the composition of the present invention is also used to (a) reduce food intake; (b) reduce body weight; (c) reduce drinking water; (d) reduce fat content; (e) improve obesity-related indicators; (f) improve metabolism-related indicators; (g) regulate the expression of lipid metabolism genes; (h) promote the utilization of fat; (i) reduce fat storage and synthesis in adipose tissue; and / or (j) increase lipolysis in adipose tissue.

In general, the active ingredients of the present invention can be formulated in a non-toxic, inert and pharmaceutically acceptable carrier medium. The formulated pharmaceutical composition can be administered by conventional routes, including (but not limited to): oral, intramuscular, intraperitoneal, intravenous, subcutaneous, intracutaneous, or topical administration.

The present invention also provides a pharmaceutical composition, which contains a safe and effective amount of the active ingredient of the present invention and a pharmaceutically acceptable carrier or excipient. Such carriers include (but are not limited to): saline, buffer, glucose, water, glycerin, ethanol, and combinations thereof. The pharmaceutical preparation should match the mode of administration. The pharmaceutical composition of the present invention can be prepared in the form of an injection, for example, prepared by a conventional method using physiological saline or an aqueous solution containing glucose and other adjuvants. Pharmaceutical compositions such as tablets and capsules can be prepared by conventional methods. Pharmaceutical compositions such as injections, solutions, tablets and capsules should be manufactured under sterile conditions. The administration amount of the active ingredient is a therapeutically effective amount, for example, about 1 microgram to 10 milligrams per kilogram of body weight per day. Preferably, the dosage of Licoricone or its derivatives may be: 0.1 to 2000 mg per day for adults, preferably 1 to 300mg / day.

As a medicine for treating or preventing obesity or a related disease thereof, it can be made into oral and non-oral preparations. Oral administration can be made into common dosage forms such as tablets, powders, granules, capsules, etc. The excipients used can be one or more of starch, lactose, sucrose, mannose, hydroxymethyl cellulose and the like. The disintegrant may be one or more of potato starch, hydroxymethyl cellulose and the like. The bonding agent may be one or more of gum arabic, corn starch, gelatin, dextrin, etc. In addition to the above dosage forms, oral preparations can also be prepared as emulsions, syrups, and the like.

Non-oral preparations can be made into injections, and can be made into injections with water for injection, physiological saline, and glucose water, and a certain proportion of ethanol, propanol, ethylene glycol, etc. can be added to them.

A further object of the present invention is to provide a method for preparing a medicament for treating or preventing obesity or a related disease thereof, using the compound represented by formula I or a pharmaceutically acceptable salt thereof and optional other medicines for treating or preventing obesity or a related disease thereof as the raw material of medicine, and the corresponding excipients are used to prepare oral and non-oral preparations according to the conventional method, wherein the amount of the compound represented by formula I or its pharmaceutically acceptable salt can be: 0.1 to 2000 mg per day for adults, preferably 10 to 500 mg / day, taken once a day; children's dosage and frequency should be reduced on an adult basis as appropriate.

### Kit

The present invention also provides a kit, containing:
(i) a first container, and an active ingredient (a1) a compound of formula I, or a pharmaceutically acceptable salt thereof according to the first aspect of the present invention or a drug containing the active ingredient (a1) contained in the first container; and
(ii) an optional second container, and an active ingredient (a2) other drugs for treating or preventing the obesity or a related disease thereof, or a drug containing the active ingredient (a2) contained in the second container; and
(iii) instructions.

The preparation containing the compound of Formula I or a pharmaceutically acceptable salt thereof may be a unit dosage form containing the compound of formula I or a pharmaceutically acceptable salt thereof, and the preparation containing other drugs for treating or preventing obesity or a related disease thereof may be a unit dosage form containing other drugs for treating or preventing obesity or a related disease thereof.

The kit contains at least two unit dosage forms containing the compound of Formula I or a pharmaceutically acceptable salt thereof and unit dosage forms containing other medicines for treating or preventing obesity or a related disease thereof; preferably, each is 4 - 10.

As used herein, the term "unit dosage form" refers to prepare the composition into the dosage form required for single administration for the convenience of taking, including but not limited to various solid agents (such as tablets), liquid agents, capsules, slow release agent.

In addition, the other drugs for treating or preventing obesity or a related disease thereof that can be used in the kit of the present invention may be one or more, preferably, the other drugs for treating or preventing obesity or a related disease thereof may be multiple, and more preferably, it may be other pharmaceutical combinations known to those skilled in the art for the treatment or prevention of obesity or a related disease thereof.

### Method for treating or preventing obesity or its related diseases

The present invention provides a method for treating or preventing obesity or a related disease thereof, including the steps of:
(i) administering a compound of Formula I or a pharmaceutically acceptable salt thereof of the present invention, a pharmaceutical composition or a kit containing the compound of the present invention to a subject in need thereof.

In addition, the compounds of the present invention can also be used for a variety of purposes selected from the group consisting of: (a) reducing food intake; (b) reducing body weight; (c) reducing drinking water; (d) reducing fat content; (e) improving obesity -related indicators; (f) improving metabolism-related indicators; (g) regulating the expression of lipid metabolism genes; (h) promoting the utilization of fat; (i) reducing fat storage and synthesis in adipose tissue; and/or (j) increasing lipolysis of adipose tissue.

### The main advantages of the present invention include:

(a) The present invention has for the first time found a class of compounds of formula I or their pharmaceutically acceptable salts that can specifically target NUCB2 protein and can effectively treat obesity or it related diseases.
(b) The compound of Formula I screened out by the present invention or a pharmaceutically acceptable salt thereof also has a very good druggability (excellent solubility, bioavailability, etc.).
(c) The present invention has for the first time found that the compound of formula I screened by the present invention or a pharmaceutically acceptable salt thereof can (a) reduce food intake; (b) reduce body weight; (c) reduce drinking water; (d) reduce fat content; (e) improve obesity-related indicators; (f) improve metabolism-related indicators; (g) regulate the expression of lipid metabolism genes; (h) promote the utilization of fat; (i) reduce fat storage and synthesis in adipose tissue; and/or (j) increase lipolysis of adipose tissue.
(d) So far, the molecular mechanism of NUCB2 gene function is not clear. The present invention has for the first time found the small chemical molecules (such as the compound of formula I of the present invention) that bind to NUCB2 protein. As a research tool, it can also promote the progress of basic research.
(e) The present invention has for the first time found that the 83-122 amino acids of NUCB2 are the key sites for binding to the compounds of formula I of the present invention (such as HLY78).

The present invention is further described below with reference to specific embodiments. It should be understood that these examples are only for illustrating the present invention and not intended to limit the scope of the present invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions described in the Journal of Microbiology: An Experimental Handbook (edited by James Cappuccino and Natalie Sherman, Pearson Education Press) or the manufacturer's proposed conditions.

Unless otherwise stated, the materials and reagents used in the examples are all commercially available products.

### General materials and methods

### 1. Plasmids and antibodies

The full length of mouse NUCB2 gene was obtained by PCR and cloned into mammalian expression vector PCMV-HA and prokaryotic expression vector PET-28A. Nesfatin-1, Nesfatin-2, Nesfatin-3, and Nesfatin1 + fragments were cloned in PET-28A. NUCB2 (N6789) antibody was purchased from Sigma Aldrich, HA (16B12) antibody was purchased from Covance Corporation, and His antibody was purchased from Sigma Aldrich. Chemically modified cyclodextrin Captisol (HY-17031) was purchased from MCE Corporation. HLY72, HLY78, HLY103, HSS49A, HLY179 chemical small molecules come from Mr. Hao Xiaojiang's Laboratory, Kunming Institute of Botany, Chinese Academy of Sciences.

### 2. Protein expression and purification

Wnt3a protein was prepared from Drosophila S2 cell culture fluid, prepared through a two-step purification method of Blue Sepharose cation exchange column and molecular sieve, and the titer was determined before use to determine the dilution ratio during use.

The full length and fragment of NUCB2 were expressed in E. coli BL21-CodonPlus (DE3). After shaking at 37 °C to an OD value of about 0.8, it was cooled at 20 °Cfor half an hour. IPTG was added to a final concentration of 500 µM, and the bacteria were harvested after induction at 20 °Cfor 18 hours. The bacterial solution was resuspended in lysis buffer (50 mM HEPES pH 7.4, 1 mM MgCl₂, 1 mM CaCl₂, 5 mM imidazole, EDTA-free cOmplete Protease Inhibitor Cocktail), and the bacteria were broken with a bacteria breaker, it was centrifuged at 8000 g for 30 minutes at 4 °C, and supernatant was combined with Ni-NTA column for 1 hour. It was eluted with 400 mM imidazole, the buffer was concentrated and changed in 50 mM HEPES pH 7.4.

### 3. Cell culture and transfection

HEK293T cells were cultured in DMEM (Invitrogen) medium containing 10% fetal bovine serum at 37 °C with a CO₂ concentration of 5%. Cells were divided at 18-24 hours before transfection. The total plasmid volume of the 6-well plate was 2 µg / well. When the amount of transfected plasmid was insufficient, make up with LacZ. The transfection reagent was Lipo3000 (Invitrogen). The corresponding experiment was done at 24 hours after transfection.

### 4. Reporter live test

The HEK293T cells used for the measurement of reporter gene activity were plated at 20 hours before transfection and cultured to about 50% coverage for the transfection. The transfection method was as described above. For HEK293T cells, transfection was performed according to 20 ng TOPFlash, 20 ng GFP and 460 ng lacZ per well, and Wnt3a or Nesfatin-1 / NUCB2 protein was added at 18 hours after transfection to stimulate for 6-8 hours, and the cell was lysed with Boehringer Mannheim Luciferase Assay Kit (200 µ 1 / well) for 15 minutes, 50 µl lysate was added in the fluorometer BIO-TEK Synergy2 (Gene Company Limited) to measure the GFP fluorescence intensity in the cell lysate as an internal standard, and then 20 µl luciferase substrate (Roche) was added for the reaction, the luciferase activity was detected in the chemiluminescence channel, and finally the GFP reading was used as an internal standard for homogenization.

### 5. In vitro binding experiment and Western blot for biotinylated HLY78

When doing the combining experiments, (1) pre-treating Streptavidin-Agarose, washing Streptavidin-Agarose Beads with IP buffer, and spinning it on the rotor for 10 minutes. (2) Re-binding small molecules HLY179 (20µM) and Streptavidin-Agarose Beads, spinning it in a refrigeration house at 4 °C for 1-2 hours. (3) When the previous step was performed for about 1 hour, the protein was pre-cleared at the same time, and the protein and the washed Streptavidin-Agarose were rotated in a refrigeration house at 4 °C for 30 minutes. (4) the Streptavidin-Agarose Beads binded with HLY179 was centrifuged, the supernatant was removed, washed again with 600 µL of IP buffer (rotated at 4 °Cfor 3 minutes), centrifuged, and the supernatant was removed carefully. (5) the pre-cleard protein was added and rotated in a refrigeration house at 4 °C for 2 hours. Washd twice with IP buffer 800 µL, 2 minutes each time (all rotated in a refrigeration house at 4 °C). After the last wash, the supernatant was removed, 40 µL of 2 * loading was added and cooked for 10 minutes, stored at -20 °C. When doing competitive binding experiments, in the third step while pre-treating the protein adding different small molecule compounds, the final concentration was 100 µM, and the rest was the same. Western Blot: Preparing the appropriate concentration of SDS-PAGE gel, adding protein sample for electrophoretic separation. Electrotransferring the protein in the gel to the NC (nitrocellulose) membrane and blocking it with 0.5% skim milk for 1 hour. Washing three times with TBST for 5 minutes each time, then adding the corresponding primary antibody and incubate overnight at 4 °C. After recovering the antibody, it was washed with TBST 3 times for 5 minutes each time, and the corresponding HRP-conjugated secondary antibody was added, and incubated for 1 hour at room temperature. After washing three times, adding developing solution to scan. For the proteins Nesfatin1 and 2 with small molecular weight, the Tricine-SDS-PAGE method was used, and the process was consistent with the literature.

### 6 Small molecule and NUCB2 protein binding experiment

When doing the binding experiments, (1) pre-treating Streptavidin-Agarose, washing Streptavidin-Agarose Beads with IP buffer, and it was rotated on the rotor for 10 minutes. (2) Re-binding small molecules HLY179 (20µM) and Streptavidin-Agarose Beads, rotated in a refrigeration house at 4 °C for 1-2 hours. (3) When the previous step was performed for about 1 hour, the protein was pre-cleared at the same time, and the protein and the washed Streptavidin-Agarose were rotated in a refrigeration house at 4 °C for 30 minutes. (4) the Streptavidin-Agarose Beads binded with HLY179 were centrifuged, the supernatant was removed, washed once with 600 µL of IP buffer (rotated at 4 °C for 3 minutes), centrifuged, and the supernatant was removed carefully. (5) Adding the pre-cleared protein and it was rotated in a refrigeration house at 4 °C for 2 hours. It was washed twice with IP buffer 800 µL, 2 minutes each time (all rotated in a refrigeration house at 4 °C). After the last wash, the supernatant was removed, 40 µL of 2 * loading was added and cooked for 10 minutes, stored at -20 °C. When doing competitive binding experiments, in the third step while pre-treating the protein adding different small molecule compounds at a final concentration of 100 µM, and the rest was the same.

### 7 Western Blot

SDS-PAGE gel with appropriate concentration was prepared and protein sample was added for electrophoretic separation. Electrotransferring the protein in the gel to the NC (nitrocellulose) membrane and blocking it with 0.5% skim milk for 1 hour. It was washed three times with TBST for 5 minutes each time, then the corresponding primary antibody was added and incubated overnight at 4 °C. After recycling the antibody, it was washed with TBST 3 times for 5 minutes each time, and the corresponding HRP-conjugated secondary antibody was added, and incubated for 1 hour at room temperature. After washing three times, the developing solution was added to scan. For the proteins Nesfatin1 and 2 with small molecular weight, the Tricine-SDS-PAGE method was used, and the process was consistent with the literature.

### 8 mouse

All animal experiments were approved by the Animal Management Committee of SIBCB. C57BL / 6 mice were purchased from Lingchang company (normal mice and high-fat diet-induced obese mice), C57 / BLJ6 (NUCB2 gene knockout mice for breeding and background purification) were purchased from Lingchang company, OB / OB mice were purchased from Slack company. A high-fat diet (45% kcal, D12451) for inducing obese mice was purchased from Research Diets. Starting from the sixth week, it was fed with high-fat diet for 14-25 weeks. The rest of the mice used normal diet (13.5% kcal). The mice were housed in the SPF grade animal house of SIBCB. The lights were turned off for 12 hours (19: 00-07: 00), and the lights were turned on for 12 hours (07: 00-19: 00).

### 9 Determination of food intake, body weight and body fat

Mice were intraperitoneally injected with DMSO or HLY72 (dissolved in DMSO) 25 µL within 2 hours before turning off the lamp. During the intragastric experiment, the high concentration of HLY72 dissolved in DMSO was dissolved in 10% cyclodextrin, and the total volume was 100 µL Volume by gavage, so that the final concentration of DMSO was less than 10%. The diet was weighed at the 3rd hour, the 6th hour, the 12th hour, and the 24th hour respectively after the lights out. The corresponding mouse body weight was also weighed at the same time before the daily administration. The fat content of mice was measured with the magnetic resonance fat content measuring instrument of the animal platform in SIBCB.

### 10 Metabolic cage

Before starting the metabolic cage experiment, the mice were fed in a single cage for 1 week. After a one-week single cage adaptation period, three days before the start of the small molecule compound injection, the mice were injected with 25 µL of DMSO within 2 hours before turning off the lights every afternoon, allowing them to adapt to the injection for 3 days. After 3 days of injection, the acclimated mice were put into the metabolic cage. Starting from the same day, 25 µL of DMSO and HLY72 (20 mg / kg) were injected within 2 hours before turning off the lights for 3 consecutive days. When analyzing the data, the data on the first night was not included for analysis, followed by two consecutive days (daytime on the next day, daytime on the third day), two nights (the second night, the third night), the 48 hours of data respectively were used as the final data displayed by the metabolic cage.

### 11 RNA extraction and RT-PCR in the tissue and cell

After the mice were euthanized, the corresponding tissues were immediately peeled off and quickly frozen with liquid nitrogen and stored at -80 °C. For the cell sample in the 6-well petri dish, after removing the culture medium, 1 mL of Trizol was added and placed at room temperature for 10 minutes, then transferred to a 1.5 mL EP tube and stored at -80 °C. When extracting tissue RNA, 1.5 mL of Trizol was added to the tissue homogenization tube containing steel balls, the tissue was removed from the -80 °C refrigerator and put into the homogenization tube to homogenize and crush. RNA was extracted by phenol-chloroform-isopropanol method. Using the superscriptTMIII first strand synthesis system (Invitrogen) kit, 1 µg of mRNA was reverse transcribed into cDNA, and random primers were used for reverse transcription. Quantitative SYBR green PCR kit (Takara) kit was used for quantitative PCR reaction. The instrument for PCR reaction was ABI-QuantStudio 6 Realtime PCR instrument.

### 12 Paraffin section of mouse liver tissue

Mouse liver tissues were taken and fixed in 4% paraformaldehyde at 4 °C overnight. It was washed twice with PBS buffer solution in 30% and 50% ethanol for 30 minutes each, and stored in 70% ethanol at 4 °C. (1) Gradient dehydration of ethanol: 80% ethanol for 30 minutes, 95% ethanol for 30 minutes, 100% ethanol for 15 minutes, and fresh 100% ethanol for 15 minutes. (2) Xylene transparent: 1/2 absolute ethanol plus 1/2 xylene for 15 minutes, xylene for 10 minutes, and fresh xylene for 10 minutes. (3) Wax immersion, embedding, sectioning: 1/2 xylene plus 1/2 paraffin for 30 minutes, paraffin 1 for 90 minutes, paraffin 2 for 120 minutes, paraffin 3 overnight for embedding, conventional section thickness was 4 µm, pasted on clean glass slides that have been processed, the slides were baked at 37 °C overnight, collected in a slide box and sealed at 4 °C. (4) Xylene dewaxing, gradient ethanol rehydration: tissue sections were dewaxed three times with xylene, 10 minutes each time, and placed in 1/2 xylene plus 1/2 absolute ethanol for 15 minutes. After 5 minutes each of 100%, 90%, 80%, 70%, 50% and 30% ethanol, it was put in pure water for 5 minutes. (5) HE staining, filming: hematoxylin staining at room temperature for 20 minutes, rinsed under running water, color separation with 0.1% hydrochloric acid and ethanol for 1 second, rinsed under running water, after 1 minute each of 30%, 50%, 70%, 80% and 95% ethanol, it was stained with 1% eosin for 1 minute. It was put in absolute ethanol for 1 minute and dehydrated with fresh absolute ethanol twice for 5 minutes each time, it was put in 1/2 absolute ethanol plus 1/2 xylene for 15 minutes, and then xylene 1,2,3 for 5 minutes each time. Sealed with neutral gum and stored at room temperature in a dry place. The cell platform Olympus BX51 microscope was used for filming.

### 13 Statistical analysis of data

The data is presented as mean ± standard error (means ± s.e.m). The P value was obtained by Student's test, and the significance was expressed as *: P < 0.05, **: P < 0.01, ***: P < 0.001.

### Example 1 An interaction between the small molecule compound HLY78 and NUCB2

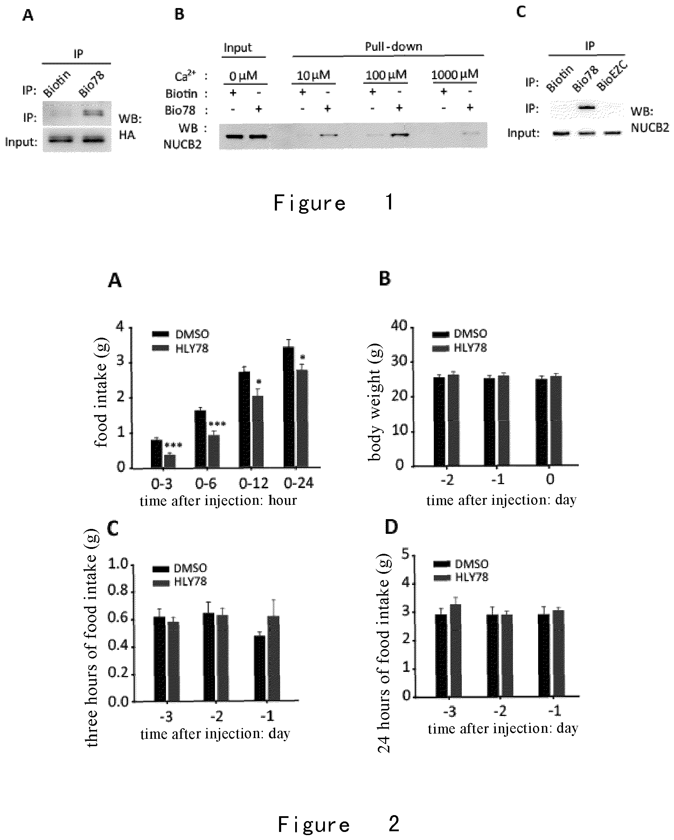

Mass spectrometry identification shows that there may be an interaction between the small molecule compound HLY78 and NUCB2. To verify this speculation, first, the mouse NUCB2 gene was cloned, and in vitro binding experiments was performed using HEK 293T cells overexpressed HA-tagged full-length NUCB2 protein and Bio78 (Biotin-linked HLY78). The results show that Bio78 can bind to the NUCB2 protein expressed by cells (Figure 1a).

Immediately the mouse full-length NUCB2 protein expressed in E. coli was purified. When analyzing the characteristics of NUCB2 protein, it is found that it contains two EF-hand domains, suggesting that it is a Ca²⁺ binding protein. Therefore, different Ca²⁺ concentrations were used in the experiments. The results show that neither too high nor too low Ca²⁺ is not a good condition. When the concentration of Ca²⁺ is 100 µM, NUCB2 may have a better conformation and exhibit better binding capacity with small molecules (Figure 1b).

In order to further understand the specificity of the binding of HLY78 to the NUCB2 protein, another small molecule EZC (EZC is the code name of the small molecule, like HLY78, EZC also has Bio-EZC with Biotin connected) under investigation in the laboratory was selected for this purpose. Binding experiments reveal that the purified NUCB2 protein specifically binds HLY78 but not EZC (Figure 1c).

### Example 2 HLY78 reduces the food intake of mice

The next question we are very concerned about is whether HLY78 has similar functions to NUCB2 protein in mice. Up to now, the most classic function reported for NUCB2 protein is to reduce the food intake of mice at night. Therefore, the experiment was designed: 20 mg / kg of HLY78 was injected intraperitoneally within two hours before turning off the lights at night, and then the food intake of the mice was measured at different time points (0-3 h, 0-6 h, 0-12 h, 0 -24 h). The results clearly show that HLY78 does significantly reduce the food intake of mice (Figure 2a). During the three days of the adaptive injection period before HLY78 injection, both groups of mice were injected with DMSO. On the day of injection of HLY78 and the two days before the injection of DMSO, the body weight of the two groups of mice is also the same (Figure 2b). Statistics on the feeding data of the two groups of mice during the 3 days show that there is no significant difference in the eating conditions of the two groups of mice, including 0-3 hours and 0-24 hours (Figure 2c, 2d).

### Example 3 HLY78 analog HLY72 competes for the binding of HLY78 to NUCB2

Given that HLY78 can synergistically activate the Wnt signaling pathway, and the activity of the Wnt signaling pathway is related to many biological processes, such as the differentiation of adipocytes and some neural activities. Two reasons: (1) In order to simplify the complexity of the research, (2) in order to find the key group that HLY78 plays its role in reducing food intake, we decide to find small molecules that can bind to NUCB2 but do not activate Wnt signal from the derivatives of HLY78 as new target small molecules. We focus on the three small molecules HLY72, HLY103, and HSS49A (Figure 3a). Because the structures of these molecules are most similar to HLY78, in addition, none of them have the activity of Wnt signal activation (Figure 3b). So that the first biochemical experiment design is: whether these three small molecules can compete for the binding of HLY179 to NUCB2 protein. The results show that HLY78 can compete with Bio78 for binding to NUCB2, HLY72 can also compete with Bio78 for binding to NUCB2, while HLY103 and HSS49A do not have this competitive ability (Figure 3c).

### Example 4 HLY72 reduces the food intake of mice

Next, two small molecules HLY72 and HLY103 were selected to perform functional experiments on the food intake in mice. Completely consistent with the biochemical combination experiment, 20 mg / kg of HLY72 has a significant effect on inhibiting food intake (0-3 h, 0-6 h, 0-12 h, 0-24 h), while HLY103 has no effect (Figure 4a) . The body weight of the two groups of mice is the same two days before and on the day of administration (Figure 4b), and in the three days of the adaptive injection period before HLY72 injection, the statistics of the two groups of mice injected with DMSO show that there is no difference in the eating conditions of mice (Figure 4c, 4d). Subsequently, using three concentrations of HLY72, it is found that the effect of HLY72 on reducing the food intake of mice shows a dose gradient. At 7.5 mg / kg, HLY72 has no significant effect on the food intake of mice, and shows a significant gradient effect at 15 mg / kg and 30 mg / kg (Figure 4e, 4f).

Therefore, through the biochemical competitive binding experiments and functional tests of food intake, HLY72 is re-established as a small molecule compound targeting NUCB2 to treat obesity. At the same time, through these experiments, we have preliminarily determined that the key group for HLY72 to perform related functions is the site near the nitrogen atom.

### Example 5 Intraperitoneal injection of HLY72 can reduce the weight of normal mice

After screening better small-molecule compound and probably determining its key groups, whether HLY72 has a weight-loss effect has become the most wanted result immediately. For this problem, we directly used normal mice intraperitoneally injected with HLY72 for 5 consecutive days. The results show that 20 mg / kg of HLY72 can significantly reduce the body weight of normal mice, from 25.73 ± 0.29 g to 23.37 ± 0.36 g (Figure 5a), corresponding to a reduction in total body weight of 9.21% ± 0.74% (Figure 5b), At the same time compared with the control group, the daily food intake is also significantly reduced in HLY72 group (Figure 5c).

### Example 6 Oral gavage of HLY72 can also reduce the weight of normal mice

In order to further investigate whether the administration of HLY72 by oral gavage also has a weight-loss effect, and because of the convenience of oral administration, many drugs are taken orally. We have also considered one step more for future applications, so that the administration of oral gavage was used to see the effect of HLY72 on the body weight of normal mice. The same was 20 mg / kg HLY72 intragastric treatment for 5 days. The body weight of mice is decreased from 25.26 ± 0.37 g to 23.14 ± 0.45 g (Figure 6a), corresponding to a reduction in total body weight of 8.43% ± 0.81% (Figure 6b), compared with the control group, the daily food intake of mice in the HLY72 group is also decreased significantly (Figure 6c).

### Example 7 HLY72 specifically reduces the weight of mice while HLY103 cannot

After confirming that HLY72 can indeed reduce the weight of mice, we examine two more questions: (1) Whether HLY72's weight-reducing effect is dose-dependent; (2) Whether HLY72-like molecule HLY103 not binding to NUCB2 has a weight-reducing effect. The experiment for 3 consecutive days clearly shows that the effect of HLY72 on weight loss is a concentration gradient (Figure 7a), while HLY103 has no effect on body weight at all (Figure 7b). We also do the statistics on the daily food intake of the mice in these three days, which is consistent with the conclusion of the short-term statistics in the previous day.

### Example 8 HLY72 has a good weight-loss effect on OB / OB obese model mice

After obtaining the above results, we now begin to use the small molecule HLY72 in obese mouse models. We first chose the obese mouse model (OB / OB) caused by Leptin mutation. An important reason for the persistence of obesity is the resistance and tolerance to regulatory factors such as Insulin and Leptin. And we are very pleased with the fact that NUCB2 can reduce the food intake of Leptin receptor mutant Zucker rats. In this regard, we can reasonably speculate that the small molecule HLY72 should also have an effect on OB / OB mice. Sure enough, the injection of HLY72 has a significant weight loss effect on OB / OB mice. With 20 mg / kg of HLY72 injection for 7 consecutive days, the body weight of OB / OB mice is decreased from 42.02 ± 1.01 g to 37.73 ± 0.87 g (Figure 8a), corresponding to a 10.15% ± 0.87% reduction in total body weight (Figure 8b). Compared with the DMSO group, the daily food intake of OB / OB mice is also significantly reduced in the HLY72 group (Figure 8c).

### Example 9 Effect of HLY72 on drinking water in mice

Since OB / OB mice are a diabetic mouse model, they have the characteristics of polyphagia, polydipsia, and polyuria. During the OB / OB mouse experiment, we also noticed a phenomenon: the urine of mice injected with HLY72 was significantly reduced. Again whether HLY72 had the effect of reducing drinking water in mice was tested. By injecting 20 mg / kg HLY72 for three consecutive days, the experimental data show that for the mice of normal chow diet (NCD), the three-day water consumption is only 39.68% ± 2.29% of the control group. For the mice of high-fat diet (HFD), the three-day water consumption is only 26.38% ± 2.06% of the control group (Figure 9). Therefore, HLY72, like NUCB2 / Nesfatin-1, has the effect of reducing water consumption in mice.

### Example 10 The effect of HLY72 on weight loss in high fat induced obesity model

According to the previous results, it is considered that HLY72 should not be used continuously for a long time. In view of the fact that many mechanisms of NUCB2 functioning are still unclear, the simplest intermittent drug administration method was selected, that is, the drug was administered for 3 days, stopped for 4 days, one dosing cycle per week, and repeated for 3 courses (Figure 10a).

In the high-fat-induced obese mouse model, three days after the first injection of HLY72, the body weight of the mice is decreased from 33.56 ± 0.63 g to 31.61 ± 0.74 g, and three days after the second administration, the body weight of mice is decreased from 31.23 ± 0.57 g to 29.36 ± 0.58 g, three days after the third administration, the body weight of mice is decreased from 29.39 ± 0.51 g to 27.80 ± 0.62 g, at the end of 21 days after 3 cycles, the overall weight of the mice is decreased from 33.56 ± 0.63 g to 27.65 ± 0.54 g (Figure 10b). The whole process corresponds to 16.88% ± 0.51% of the total weight loss (Figure 10c). Calculating the average daily food intake of the mice in the three administration time periods, compared with the DMSO group, the HLY72 group has a significant decrease (Figure 10d). Therefore, the above results clearly show that HLY72 molecule has a good weight loss effect on the two pre-clinical obese mouse models.

### Example 11 HLY72 reduces fat content

In the experiment, high-fat diets were used to feed obese mice weighing about 40 grams for 22 weeks, according to the way used in the previous 3 courses. First, four courses of HLY72 injection significantly reduce the weight of mice (Figure 11a). The analysis of the MRI fat content measuring instrument also shows that the fat content is significantly reduced (Figure 11b), and at the same time, the proportion of muscle is increased by about 9 percentage points (Figure 11c).

For normal mice, whether it is intraperitoneal injection (Figure 11d, 11e) or oral gavage (Figure 11f, 11g), after 5 consecutive days of treatment, the fat content of the mice is decreased significantly, while the muscle content remains unchanged.

### Example 12 HLY72 improves various indicators of obesity

The effects on the various adverse indicators of obesity after drug administration were detected. Through blood biochemical tests, the blood glucose of mice in the HLY72 group is also decreased significantly (Figure 12a), triacylglycerol (Figure 12b), and cholesterol levels are also decreased significantly (Figure 12c), indicating that glucose and lipid metabolism-related abnormalities have been improved. At the same time, alanine transaminase ALT is decreased (Figure 12d), aspertate aminotransferase AST does not change (Figure 12e), indicating that after using HLY72, the liver function of mice has bee promoted and improved. The liver slices of mice also clearly show that fatty liver caused by high fat is well treated (Figure 12f).

### Example 13 Effect of HLY72 on metabolism

Next, the effect of HLY72 on the metabolism of mice is studied. We use two methods: (1) the relevant metabolic indexes of the mice are directly measured using the metabolic cage, and (2) the expression of the relevant key metabolic genes is detected. three metabolic parameters are obtained through the metabolic cage experiment: (1) Energy expenditure (EE), energy output is an important aspect of energy balance, under conditions such as restricted diet and fasting, mice will reduce basic metabolism, reduce energy expenditure; (2) respiratory exchange ratio (RER), based on the ratio calculated by CO2 produced and the use of 02, RER of 1 means that carbohydrates is mainly used as energy, RER of 0.7 means that fat is mainly used as fuel; (3) physical activity (Activity).

For normal-fed mice (Lean), HLY72 treatment significantly reduces energy expenditure in mice at night (dark) and during the day (light) (Figure 13a). The respiratory exchange rate is significantly reduced at night in the HLY72 treatment group, and there is no change during the day, indicating that the experimental group of mice increases the energy utilization mode using fat as energy at night (Figure 13b). Physical activity analysis shows that the night activity in the HLY72 treatment group is decreased, which does not affect the daytime activity. It is likely to reduce the food-seeking behavior at night (Figure 13c), so that the energy expenditure at night is also reduced.

For obese mice fed with high-fat diet (DIO), the energy expenditure in the HLY72 group is significantly reduced at night and during the day (Figure 13d). Compared with the DMSO group, the respiratory exchange rate is significantly reduced at night and during the day in the HLY72 group, indicating that the mice in the experimental group increases the energy utilization method using fat as energy (Figure 13e). The physical activity is significantly reduced at night, but there is no change during the day (Figure 13f).

### Example 14 Paired feeding experiment

Next, the HLY72 injection group and their paired feeding group are compared. The DMSO-injected mice were divided into two groups. The first group was free to get enough food, while the other group was provided the same amount of food as the HLY72 group daily. The results show that the daily feed intake of the HLY72 group and its paired feeding group are almost the same (Figure 14a). Compared with the DMSO group, the energy expenditure of the HLY72 group and its paired feeding group are decreased day and night. There is no difference in energy expenditure between the HLY72 group and its paired feeding group during the day and night (Figure 14b). Compared with the paired feeding group and the DMSO treatment group, the night and day respiratory exchange rates of the HLY72 group are significantly reduced, and the paired feeding group and the DMSO treatment group also show a significant difference during the day and night (Figure 14c). In terms of physical activity, it is significantly reduced in the HLY72 group than the DMSO group and the paired feeding group, but there is no significant difference between the DMSO group and the paired feeding group (Figure 14d). The body weight of the HLY72 group and the paired feeding group is decreased significantly, but there is no significant difference between the two groups (Figure 14e).

### Example 15 Effect of HLY72 on lipid metabolism gene expression

After injection of HLY72 in mice, the epididymal white adipose tissue, liver and muscle tissue were taken to detect the expression of lipid metabolism related genes. The results are shown in white adipose tissue (Figure 15a): (1) The gene for fat storage DGAT is significantly reduced, indicating that HLY72 reduces fat storage in white adipose tissue. (2) Genes for the synthesis of fatty acids FAS and SCD1 are significantly down-regulated, indicating that HLY72 reduces fatty acid synthesis in white adipose tissue. (3) The increase in the lipolytic gene ATGL indicates that HLY72 increases the breakdown of white fat. The expression of fatty acid transporter gene FAT (CD36), FATP2, FATP5 on the liver cell membrane is increased, the expression of the transporter gene CPT1A for fatty acid entering the mitochondria and the key enzyme gene ACADM of fatty acid β-oxidation are increased (Figure 15b). At the same time, the expression of muscle cell membrane fatty acid transporter genes FATP1 and FATP4 are increased, and the expression of the transporter gene CPT1B for fatty acid entering the mitochondria and the key enzyme gene ACADM of fatty acid β-oxidation are increased (Figure 15c). Changes in gene expression in these tissues indicate that liver and muscle tissues have increased the use of fatty acids.

### Example 16 The 83-122 amino acids of NUCB2 are the key sites for HLY78 binding

In order to find the region where small molecules bind to NUCB2 protein, in vitro binding experiment (pull-down) method was used. Referring to the previous way of segmenting NUCB2, we also divided the mouse NUCB2 protein into three fragments such as Nesfatin-1 (amino acids 1-82), Nesfatin-2 (amino acids 77-171) and Nesfatin-3 (amino acids 161-196) (Figure 16a).

Subsequently, these fragments were expressed and purified using E. coli prokaryotic expression system, and Bio78 and these fragments were used for in vitro binding experiments. The results show that Nesfatin-2, the second fragment of the NUCB2 protein, mainly binds to Bio78 (Figure 16b). However, according to structural prediction and sequence analysis, it is found that 1-122 amino acids of NUCB2 protein may be a relatively complete structure, so that we constructed this fragment of 1-122 amino acids of NUCB2 and named it Nesfatin-1 +. Binding experiments show that Nesfatin-1 + has a better binding capacity to Bio78 than Nesfatin-2 (Figure 16c). According to the above results, Nesfatin-1 does not bind to Bio78, Nesfatin-2 binds to Bio78, and Nesfatin-1+ has the strongest binding to Bio78. We analyze that the 40 amino acids at positions 83-122 may be the key region where the NUCB2 protein binds to Bio78 (Figure 16d).

### Example 17 The effect of HLY72 on NUCB2 gene knockout heterozygous mice is weakened

Although the previous series of results indicate that the interactions between the small molecules HLY72 and NUCB2 are completely consistent: (1) small molecules are directly combined with NUCB2, (2) all have the effect of reducing the food intake of mice, (3) can reduce drinking water, (4) can have weight loss effect on OB / OB mice. In order to provide clear direct evidence to prove that HLY72 exerts these functions by targeting NUCB2, NUCB2 gene knockout mice were constructed for experiments.

Heterozygous mice prepared by the CRISPR-Cas9 system were purchased from Xiamen University. The specific method was to design two sgRNAs in the first exon E3 (Exon3, the first two exons E1 and E2 do not contain the protein coding sequence) of the NUCB2 gene translated into protein (Figure 17a). Heterozygous mice deleted 83 bases in E3 and confirmed by sequencing (Figure 17c). In order to facilitate the experiment, for mice with 83 bases deleted, we designed primers to detect by PCR (Figure 17b). Because of the failure to obtain NUCB2 knockout homozygous mice for multiple matches, only heterozygous mice can be used for research. Experiments show that the effect of HLY72 on heterozygous mice is reduced, whether it is daily food intake (Figure 17d) or mouse weight (Figure 17e), this result also means that HLY72 functions by targeting NUCB2.

### Example 18

HLY72 and HLY78 are potential small molecule compounds for the treatment and prevention of obesity, mainly by reducing food intake and increasing the use of fat. In order to determine the important groups of these two small-molecule compounds and find molecules with better effects, the applicant synthetically screened 57 modified derivatives of HLY72 and HLY78, and found compounds with a potency of 10 times or more For the preparation process of some compounds, see Preparation of Examples 1-11.

### Preparation of Example 1

### 2-bromo-4, 5-dimethoxybenzoic acid (13):

See scheme I. 2-bromo-4,5-dimethoxybenzaldehyde (250mg, 1mmol), sodium bicarbonate (200mg) and potassium permanganate (500mg) were dissolved in water (20mL), heated and stirred for 3 hours, then extracted twice with 20 mL of dichloromethane, the organic phases were combined, washed with saturated ammonium chloride solution and saturated brine respectively, and dried over anhydrous magnesium sulfate overnight and after filtration, the excess solvent was distilled off, and the residue was purified by silica gel column chromatography to obtain compound **13** as a pale yellow solid (220 mg, yield: 85%).

### Preparation of Example 2

### 2-bromo-4, 5-dimethoxy-N-methyl-Benzenemethanamine (14):

Compound **13** (260 mg, 1 mmol) was dissolved in THF (10 mL), then DMF (0.1 mL) and sulfoxide chloride (0.5 mL, 4 mmol) were added, and the reaction solution was stirred at 50 °C for 2 hours, and the excess THF was removed under reduced pressure. The residue was added dropwise to a 30% methylamine solution (20 ml) at 5 °C, stirred continuously for 1 hour and then filtered, and the filter cake was purified by column chromatography to obtain compound **14** (204 mg, yield 75%).

### Preparation of Example 3

### 4-ethyl-5-methyl-5H-8,9-dimethoxyphenanthridin-6-one (15):

Under nitrogen protection, palladium acetate (3.0 mg, 0.013 mmol), TFPA (6.2 mg, 0.027 mmol), anhydrous potassium carbonate (72.3 mg, 0.52 mmol), compound **4** (0.1 mmol) and 2-ethyl iodobenzene (0.26 mmol) were added into a round bottom flask, anhydrous acetonitrile was added to dissolve, then added to an aqueous flask containing norbornene (26.9 mg, 0.286 mmol) and stirred at 85 °C for 6 hours. After the reaction was completed, cooled to room temperature, saturated ammonium chloride (30 mL) was added and extracted with ethyl acetate (3 × 15 mL). The organic layer was dried over anhydrous sodium sulfate and concentrated. The residue was purified by column chromatography to obtain compound **15** (22.5 mg, yield: 75%).

### Preparation of Example 4

### 4-ethyl-5-methyl-5H-8, 9-dimethoxyphenanthridin (10a):

Compound **15** (30 mg, 0.1 mmol) was dissolved in THF (5 mL), LAH (20 mg) was added at -78 °C, the reaction solution was stirred for 2 hours and quenched with water, and the mixture was extracted twice with ether (20 mL), the organic phase was washed with saturated brine and concentrated, and the residue was purified by column chromatography to obtain compound **10a** (21 mg, yield: 75%).

### Preparation of Example 5

### 4-ethyl-5-methyl-5, 6-dihydrophenanthridine-8, 9-diphenol (10):

Compound **10a** (29 mg, 0.1 mmol) was dissolved in dichloromethane. After the reaction solution was cooled to -78 °C, BBr3 (100 µL, 0.2 mmol) was added dropwise. After the reaction solution was stirred for 4 hours, it was diluted with saturated sodium bicarbonate solution (10 mL), followed by extraction with dichloromethane (15 mL) twice. The organic phase was concentrated and purified by silica gel column chromatography to obtain compound **10** (120 mg, yield: 80%).

### Preparation of Example 6

### 5-methyl-4-vinyl-5, 6-dihydro- [1, 3] dioxolo [4,5-j] phenanthridine (8):

See scheme II. lycorine (300 mg, 1 mmol) was dissolved in DMF (10 mL), methyl iodide (400 µL, 2 mmol) was added, stirred at room temperature for 12 hours, after DMF was removed from the reaction solution under reduced pressure, tert-butanol (TBA, 10 mL) and potassium tert-butoxide (PTB, 1.1 g, 10 mmol) were added, the reaction solution was heated to reflux for 4h, and then quenched with saturated ammonium chloride solution, followed by extraction twice with ether (20 mL), after the organic phase was concentrated, the residue was purified by column chromatography to obtain compound **8** (240 mg, yield: 90%).

### Preparation of Example 7

### 4-ethyl-5-methyl-5,6-dihydro-[1,3]dioxolo[4,5-j]phenanthridine (9):

Compound **8** (27 mg, 0.1 mmol) was dissolved in dichloromethane (5 mL), 10% Pb / C (30 mg) was added, stirred under a hydrogen atmosphere for 24 h, filtered, the filtrate was concentrated, and the residue was purified by column chromatography to obtain compound **9** (25 mg, yield: 95%).

### Preparation of Example 8

### 4-ethyl-5-methyl-5, 6-dihydrophenanthridine-8,9-diphenol (10):

Compound **7** (55 mg, 0.2 mmol) was dissolved in dichloromethane (10 mL). The reaction solution was cooled to -78 °C, and BBr₃ (200 µL, 0.4 mmol) was added dropwise. After the reaction solution was stirred for 10 hours and diluted with saturated sodium bicarbonate solution (50 mL), followed by extraction twice with dichloromethane (25 mL). The organic phase was concentrated and purified by silica gel column chromatography to obtain compound **10** (35.7 mg, yield: 72%).

### Preparation of Example 9

### 4-ethenyl-5-methyl-5, 6-dihydrophenanthridine-8, 9-diol (11):

Compound **8** (52 mg, 0.2 mmol) was dissolved in dichloromethane (10 mL). The reaction solution was cooled to -78 °C and BBr₃ (200 µL, 0.4 mmol) was added dropwise. After the reaction solution was stirred for 10 hours, it was diluted with saturated sodium bicarbonate solution (50 mL), followed by extraction twice with dichloromethane (25 mL). The organic phase was concentrated and purified by silica gel column chromatography to obtain the compound (33.2 mg, yield: 65%).

### Preparation of Example 10

### Alkylation of compounds 10 and 11:

Compound **10** or **11** (0.1 mmol) was dissolved in dry THF (10 mL), NaH (50 mg, 2 mmol) and haloalkane (1 mmol) were added, the reaction solution was stirred at room temperature for 24 hours, and then quenched with water (50 mL), the reaction solution was concentrated, excess THF was distilled off, and extracted twice with dichloromethane (30 mL). The organic phase was washed with saturated sodium bicarbonate solution and saturated brine and concentrated. The residue was purified by column chromatography to obtain an alkylated compounds.

### Preparation of Example 11

### Acylation of compounds 10 and 11:

Compound **10** or **11** (0.1 mmol) was added to a solution containing pyridine (3 mL), anhydride or acyl chloride 0.5 (mmol) and DMAP (30 mg) and stirred at room temperature for 20 hours. After the reaction was completed, the reaction solution was poured into an ice-water mixture (50 mL) and stirred vigorously, then extracted twice with ethyl acetate (30 mL). The organic phase was washed with saturated brine and concentrated, and the residue was purified by column chromatography to obtain an acylated compounds.

Similarly, the compounds of Table C were prepared.

**Table C**

| Numbering | Molecular Structure | Molecular weight | weight (mg) | Solubility |
|---|---|---|---|---|
| Hcj92-a | 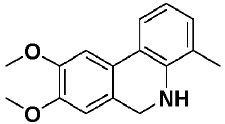 | 255 | 2.8mg | DMSO |
| Hcj12 | 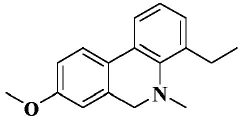 | 253 | 3.0mg | DMSO |
| | Exact Mass: 253.1467 | | | |
| Hcj 194-d | 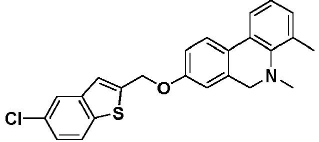 | 405 | 3.0mg | DMSO |
| Hcj 16 | 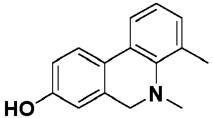 | 225 | 3.0mg | DMSO |
| | Exact Mass: 225.1154 | | | |
| Hcj46-d | 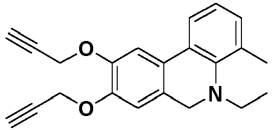 | 331 | 4.0mg | DMSO |
| | Exact Mass: 331.16 | | | |
| Hcj15 | 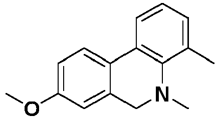 | 239 | 4.2mg | DMSO |
| | Exact Mass: 239.1310 | | | |
| Hcj 16d-a | 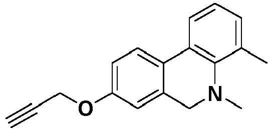 | 263 | 2.6mg | DMSO |
| Hcj44-d | 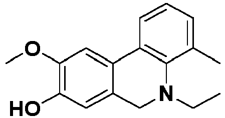 | 269 | 1.8mg | DMSO |
| | Exact Mass: 269.14 | | | |
| Hcj36 | 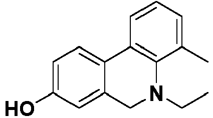 | 239 | 4.5mg | DMSO |
| | Exact Mass: 239.1310 | | | |
| Hcj 16d | 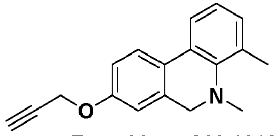 | 263 | 5.0mg | DMSO |
| | Exact Mass: 263.1310 | | | |
| Hcj221d | 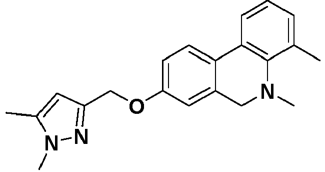 | 333 | 5.5mg | DMSO |
| | Exact Mass: 333.1841 | | | |
| Hgsy-75 | 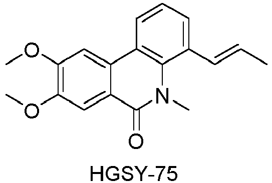 | 309.14 | 5.0mg | DMSO |
| Hgsy-78 | 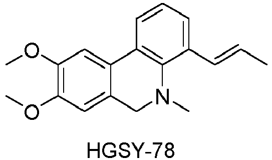 | 295.16 | 5.2mg | DMSO |
| Hgsy-80 | 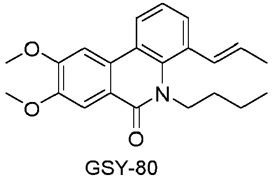 | 351.18 | 3.9mg | DMSO |
| Hgsy-86 | 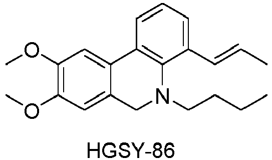 | 337.20 | 3.9mg | DMSO |
| Hgsy-89 | 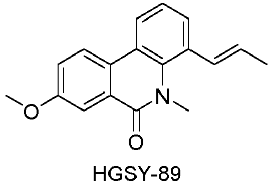 | 279.13 | 3.8mg | DMSO |
| Hgsy-92-1 | 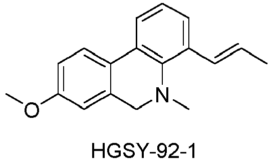 | 265.15 | 4.7mg | DMSO |
| Hgsy-97 | 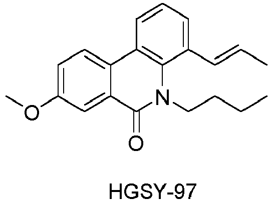 | 321.17 | 3.9mg | DMSO |
| Hgsy-56 | 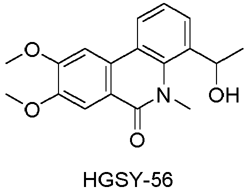 | 313 | 3.8mg | DMSO |
| Hgsy-57 | 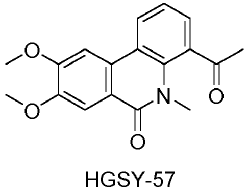 | 311 | 2.9mg | DMSO |
| Hgsy-58 | 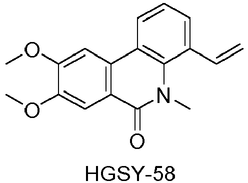 | 295 | 3.2mg | DMSO |
| Hgsy-59 | 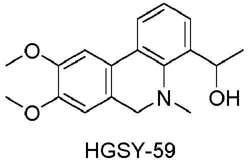 | 299 | 4.0mg | DMSO |
| Hgsy-60 | 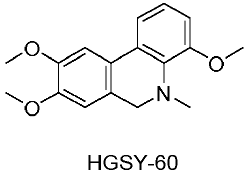 | 285 | 3.7mg | DMSO |
| Hgsy-62 | 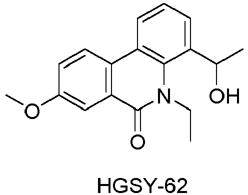 | 297 | 3.1mg | DMSO |
| Hgsy-64 | 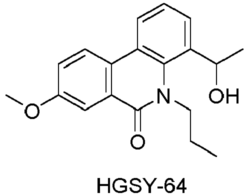 | 311 | 3.9mg | DMSO |
| Hgsy-66 | 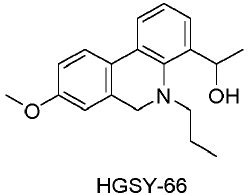 | 297 | 1.7mg | DMSO |
| Hgsy-67 | 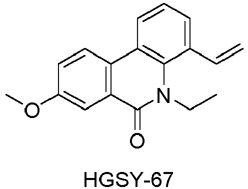 | 279 | 2.0mg | DMSO |
| Hgsy-68 | 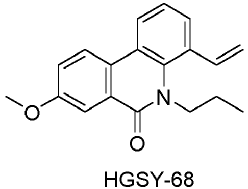 | 293 | 2.0mg | DMSO |
| Hgsy-69 | 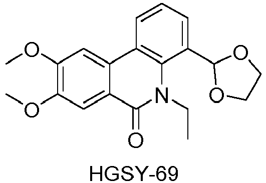 | 355 | 3.9mg | DMSO |
| Hgsy-32 | 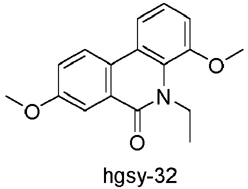 | 283 | 3.0 mg | DMSO |
| | Exact Mass: 283.12 | | | |
| Hgsy-31 | 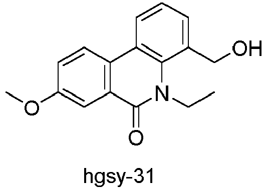 | 283 | 3.3 mg | DMSO |
| | Exact Mass: 283.12 | | | |
| Hgsy-25 | 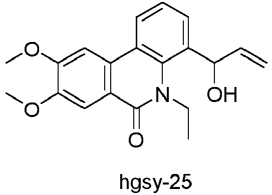 | 339 | 5.8 mg | DMSO |
| | Exact Mass: 339.15 | | | |
| Hgsy-43 | 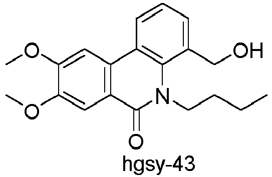 | 341 | 3.6 mg | DMSO |
| | Exact Mass: 341.16 | | | |
| Hgsy-35 | 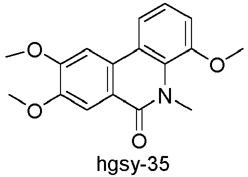 | 299 | 2.6 mg | DMSO |
| | Exact Mass: 299.12 | | | |
| Hgsy-44 | 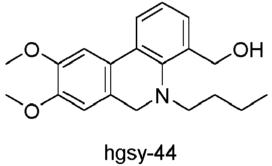 | 327 | 6.8 mg | DMSO |
| | Exact Mass: 327.18 | | | |
| Hgsy-3 | 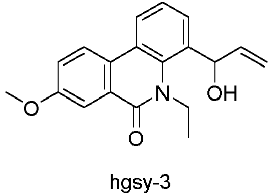 | 309 | 3.5 mg | DMSO |
| | Exact Mass: 309.14 | | | |
| Hgsy-36 | 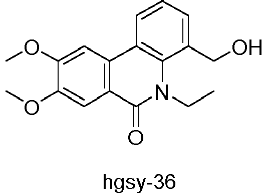 | 313 | 2.6 mg | DMSO |
| | Exact Mass: 313.1314 | | | |
| Hgsy-47 | 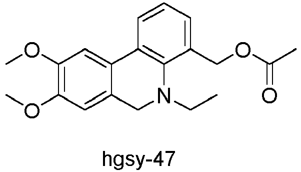 | 341 | 2.4 mg | DMSO |
| | Exact Mass: 341.16 | | | |
| Hgsy-51 | 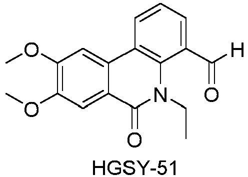 | 311 | 3.0 mg | DMSO |
| | Exact Mass: 311.12 | | | |
| Hgsy-48 | 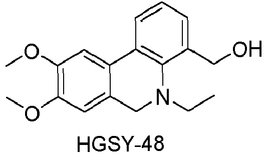 | 299 | 3.1 mg | DMSO |
| | Exact Mass: 299.15 | | | |
| Hgsy-53 | 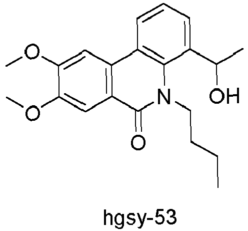 | 355 | 4.9 mg | DMSO |
| | Exact Mass: 355.18 | | | |
| Hgsy-55 | 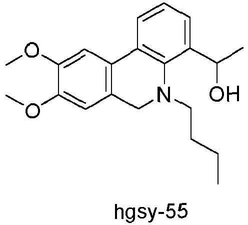 | 341 | 3.4 mg | DMSO |
| | Exact Mass: 341.20 | | | |
| Hgsy-50 | 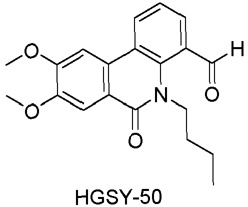 | 339 | 2.9mg | DMSO |
| | Chemical Formula: C₂₀H₂₁NO₄ Exact Mass: 339.15 | | | |
| Hgsy-98 | 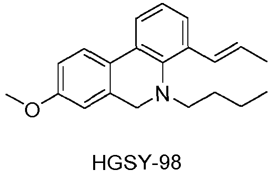 | 307.19 | 3.1mg | DMSO |
| Hybj-1a | 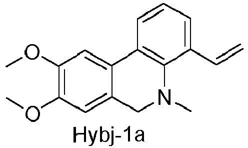 | 281 | 3.4mg | DMSO |
| Hybj-9a | 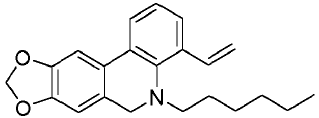 | 335 | 3.0mg | DMSO |
| | Exact Mass: 335.19 | | | |
| Hyj-12a | 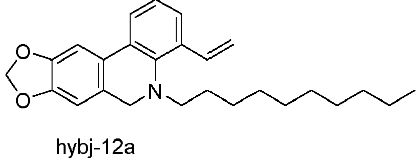 | 391 | 3.0mg | DMSO |
| | Exact Mass: 391.25 | | | |
| Hybj-13a | 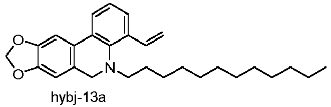 | 419 | 3.6mg | DMSO |
| | Exact Mass: 419.28 | | | |
| Hyb-17a | 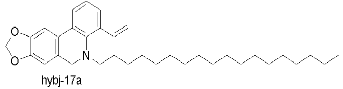 | 503 | 2.8mg | DMSO |
| | Exact Mass: 503.3763 | | | |

In addition, the following compounds were also reared:

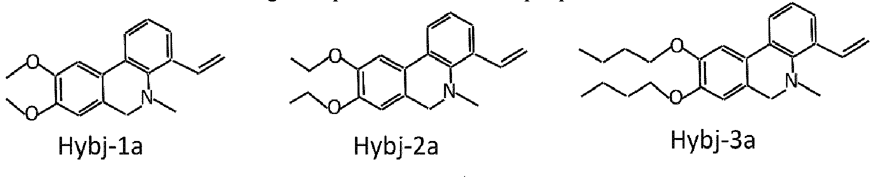

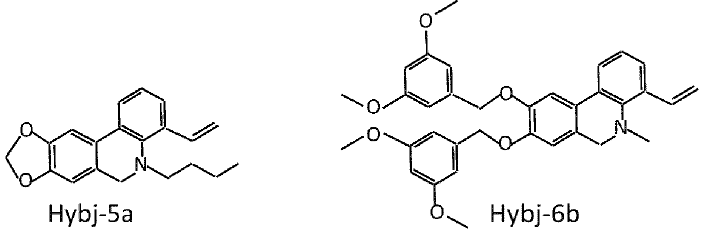

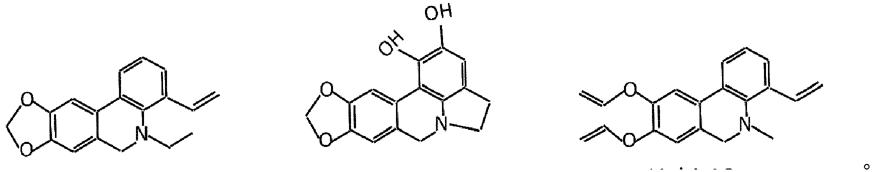

### Example 19

### Activity test

Experimental methods were the same as in Examples 2 and 4.

Experiments on the inhibition of food intake in mice show that: Hybj-2a, Hybj-3a, Hbjb-5a, Hybj-6a, Hgsy78, Hgsy80, Hgsy86, Hgsy-92-1, Hgsy35, Hgsy36, Hcj12, Hcj16, Hybj-1a, Hybj9a, Hybj12a, Hybj13a, Hybj17a, Hcj194-d, Hgsy50 have obvious effects, and the activities of compounds Hgsy-98, Hgsy-80, Hgsy-50, Hybj-12a, Hybj-13a, Hybj-17a are significantly better than HLY72..

Based on the results of the preliminary screening, in order to know more accurately the activity relationship between the compounds superior to HLY72 and HLY72. Three compounds of Hgsy-98, Hybj-12a and Hybj-13a were selected for re-experiment (5-6 mice per group).

The results are shown in Figure 18-20. The activities of Hgsy-98, Hybj-12a and Hybj-13a are high, and the effective concentration (EC₅₀) is reduced to one tenth of HLY72. It can be seen that the activity of Hgsy-98 and Hybj-13a is about 10 times that of Hly72, one order of magnitude higher. The activity of Hybj-12a is 30-40 times higher than that of Hly72.

### Conclusion

### 1. HLY78 is a small molecule that binds to NUCB2 protein and has its function

Mice experiments have shown that, similar to the function of NUCB2 in influencing the food intake of mice, HLY78 also has the function of significantly reducing the food intake in mice at night. Further screening of HLY78 chemically modified homologues, HLY72 is selected as a new small molecule targeting NUCB2. Compared with HLY78, HLY72 does not activate Wnt signal, but retains the function of binding NUCB2, and HLY72 also exhibits the function of reducing the food intake in mice at night. Therefore, the function experiment very clearly shows that HLY78 and HLY72 have the same function as NUCB2.

*In vitro* direct binding experiment shows that HLY78 is a small molecule that binds to NUCB2, and this binding is affected by calcium ions. At the same time, the experimental data of HLY78 homologues also helps us to initially determine the key group for the combination of HLY72 and NUCB and the function of reducing the food intake at night in mice, which is the part near the nitrogen atom. Although HLY179 can bind some proteins from excess NUCB2 protein by immunoprecipitation, it is difficult for us to use this sample to accurately determine the binding constant of small molecule HLY72 and NUCB2 protein. Possible reasons: (1) The purified NUCB2 protein is a mixed state composed of monomers and many different forms of polymers. Up to now, no crystal structure of NUCB2 protein has been reported so far. Zhao Jianfei, a graduate student in our laboratory, also has tried to screen a lot of crystallization conditions, but failed to get usable crystals. (2) The water solubility of HLY72 molecule is very poor. (3) The complexity of the biological significance represented by the NUCB2 gene itself. The currently known cognition may still be superficial.

### 2. HLY72 has weight loss effect on normal mice and pre-clinical obese model mice

By intraperitoneal injection and oral gavage, the small molecule HLY72 has a significant weight loss effect, and the part of weight loss determined by the magnetic resonance fat content detector is mainly adipose tissue, and the effect of reducing fat tissue is significant. As the HLY72 molecule reduces the food intake in mice, it also significantly reduces the daily water intake of mice. At present, we have initially adopted a slow and stable periodic administration method to achieve long-term administration and large scale weight loss purposes, the results show that HLY72 can significantly reduce the body weight of obese mice induced by high-fat diet. There is a need for further investigation on this issue. In addition, HLY72 molecule also has a good effect on the OB / OB obese mouse model, which is good news for leptin resistance that appears in obesity, and makes the research on the molecular and biological mechanisms of NUCB2 more urgent and attractive..

### 3. HLY72 improves metabolic and obesity related indicators

For normal-fed mice and obese mice fed with high-fat diet, the injection of HLY72 molecule significantly reduces the respiratory exchange rate, indicating that the mice in the experimental group increases the energy utilization method using fat as energy. Taking white adipose tissue to detect related gene expression, it is found that HLY72 reduces fat storage and fatty acid synthesis, and increases the breakdown of fats. Taking liver and muscle tissues to detect the expression of fatty acid utilization-related genes, it is also found that these two tissues increase the utilization of fatty acids. At the same time, blood sugar and blood lipids are significantly reduced, and liver function can be improved. All these test indicators are completely consistent, which also shows that the overall unhealthy state caused by obesity has been improved. However, whether the small molecule acts on the adipose tissue itself (periphery, because there are reports that NUCB2 has an important role in the differentiation of adipose tissue), or whether it acts on the brain (central) to regulate peripheral metabolism, or both, which is still unclear.

### 4. Outlook

The increase in obesity in the world, including developed and developing countries, cannot be directly attributed to genetic factors, because the genes of the entire human population will not change much in just a few decades. It is very easy to get food, and it is high-energy and delicious food, and changes in living conditions, such as sitting for a long time and moving less, and changes in the environment may be more important than genetics. Therefore, it is logical to reduce energy input as an important weight loss strategy through various levels, whether it is a steady-state mechanism or an unsteady-state energy control level. In the past 60 years, 25 weight-loss drugs withdrawn from the market, 23 of which act on the brain having an appetite-reducing effect, mainly on adrenal receptors, dopamine receptors, tryptamine receptors, cannabinoid receptors, etc. But on another level, these failure examples reflect a problem: many effective weight loss strategies are still by reducing energy input, and these drugs are effective mainly because they act on the central system, because the control center can better control the state of the entire individual, the effect of changing the whole through the center and the local is conceivable.

The small molecule HLY72 is a small molecule derived from the alkaloid lycorine in the bulb of the traditional Chinese medicine of *Amaryllidaceae* plant *Lycoris radiata.* Lycorine and some of its derivative modified molecules have become drug molecules for clinical use. Therefore, according to the current experimental results of the present invention, the development of HLY72 towards weight loss drugs can still be expected.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula I, or a salt thereof, or an optical isomer thereof, or a racemate thereof, or a solvate thereof, or a precursor thereof, 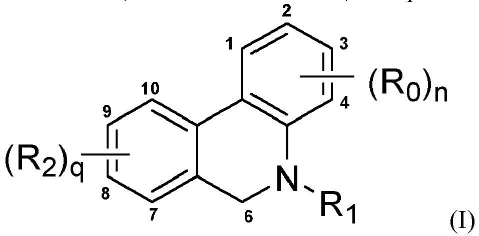 wherein,
Each R₀ is independently selected from the group consisting of: H, halogen, substituted or unsubstituted C1-C8 alkyl, substituted or unsubstituted C2-C8 alkenyl, substituted or unsubstituted C2-C8 alkynyl, substituted or unsubstituted C₃-C₈ cycloalkyl, substituted or unsubstituted C1-C8 alkoxy, R3-C (O)-, R4-C (O) -O-CH₂-, R₅ (R₆) N-, substituted or unsubstituted 5-20 membered heterocyclyl, -OH, and substituted or unsubstituted benzyl; wherein, the heterocyclyl contains 1-3 heteroatoms selected from N, O, and S, and the substituted means that H in the group is substituted by one or more substituents selected from the group consisting of: -OH, oxo (= O), -CN, nitro, carboxyl, halogen, C1-C3 alkyl, C1-C3 haloalkyl, and -NRcRd; n is 1, 2, 3 or 4, preferably 1, 2 or 3, more preferably 1 or 2;
R1 is selected from the group consisting of: H, substituted or unsubstituted C₁-C₃₀ alkyl, substituted or unsubstituted C₂-C₂₀ alkenyl, substituted or unsubstituted C₂-C₂₀ alkynyl, substituted or unsubstituted C1-C20 alkoxy, substituted or unsubstituted C₃-C₈ cycloalkyl, -OH, substituted or unsubstituted-(CH2)ₘ-C6-C12 aryl or C4-C10 heteroaryl and substituted or unsubstituted benzyl; the heteroaryl contains 1-3 heteroatoms selected from N, O, S, and the substituted means that the H in the group is substituted by one or more substituents selected from the group consisting of: halogen, -OH, oxo (= O), -CN, -NRcRd, and nitro;
Each R2 is independently selected from the group consisting of: -O-R₇, wherein R₇ is selected from the group consisting of: H, substituted or unsubstituted C1-C8 alkyl, substituted or unsubstituted C2-C8 alkenyl, substituted or unsubstituted C2-C8 alkynyl,-(CH₂)ₘ-substituted or unsubstituted C6-C12 aryl or C4-C10 heteroaryl and substituted or unsubstituted C₃-C₈ cycloalkyl; the heteroaryl contains 1 -3 heteroatoms selected from N, O, S, and the substituted means that H in the group is substituted by one or more substituents selected from the group consisting of: C1-C8 alkoxy, C1-C8 alkyl, halogen, -OH, oxo (= O), -CN, -NRcRd, and nitro; m is a positive integer of 0-6, preferably 1, 2, 3, or 4;
q is 0, 1, 2, 3 or 4;
R3 and R4 are each independently selected from the group consisting of: H, C1-C8 alkyl, C2-C8 alkenyl, C2-C8 alkynyl, C₃-C₈ cycloalkyl, halogen, -OH, -CN, -NRcRd, nitro, and a combination thereof;
R5 and R6 are each independently selected from the group consisting of: H, C1-C8 alkyl, C2-C8 alkenyl, C2-C8 alkynyl, C₃-C₈ cycloalkyl, -OH, -CN; or R5 and R6 together with the adjacent N form a 4-8 membered nitrogen-containing heterocyclic ring;
or each R2 together with the connected C atom may form a substituted or unsubstituted 5-20 membered heterocyclyl containing at least one oxygen atom, and the 5-20 membered heterocyclyl contains 1-3 heteroatoms selected from N, O, S, wherein the substituted means having one or more substituents selected from the group consisting of: C1-C8 alkoxy, C1-C8 alkyl, halogen, -OH, -CN, -NRcRd, and nitro;
Rc and Rd are each independently H, C₁-C₃ alkyl or C3-C6 cycloalkyl.

2. The compound of formula I, or a salt thereof, or an optical isomer thereof, or a racemate thereof, or a solvate thereof, or a precursor thereof, wherein the compound has a structure as shown in Formula Ia: 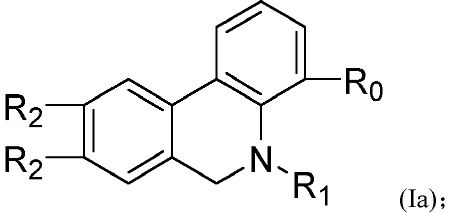 wherein, the definitions of RO, R1, and each R2 are as described above.

3. The compound of formula I, or a salt thereof, or an optical isomer thereof, or a racemate thereof, or a solvate thereof, or a precursor thereof, wherein the compound has a structure as shown in Formula Ib: 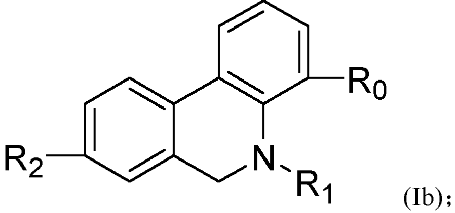 wherein, the definitions of RO, R1, and R2 are as described above.

4. The compound of formula I, or a salt thereof, or an optical isomer thereof, or a racemate thereof, or a solvate thereof, or a precursor thereof, wherein the compound has a structure as shown in Formula Ic: 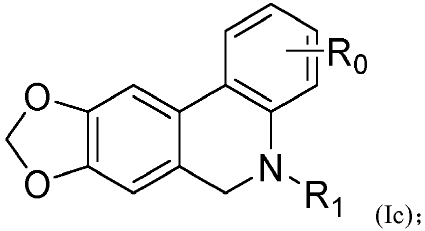 wherein, the definitions of R0 and R1 are as described above.

5. The compound of formula I, or a salt thereof, or an optical isomer thereof, or a racemate thereof, or a solvate thereof, or a precursor thereof, wherein the compound has a structure as shown in Formula Id: 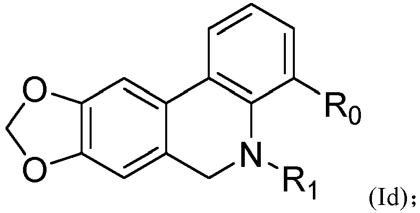 wherein, the definitions of RO and R1 are as described above.

6. The compound of formula I, or a salt thereof, or an optical isomer thereof, or a racemate thereof, or a solvate thereof, or a precursor thereof, wherein the compound is selected from the group consisting of: 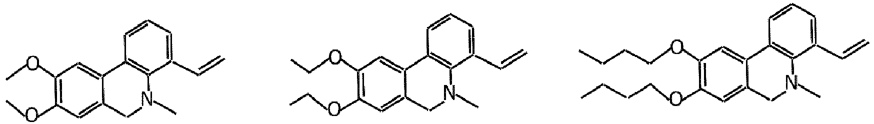 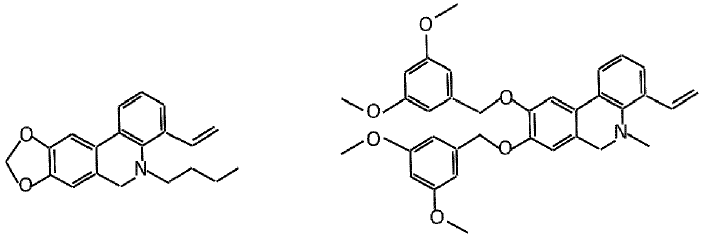 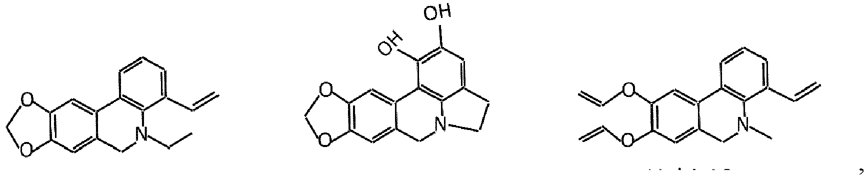 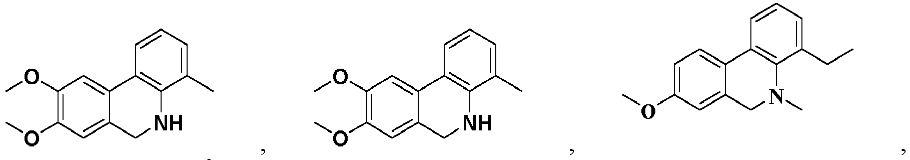 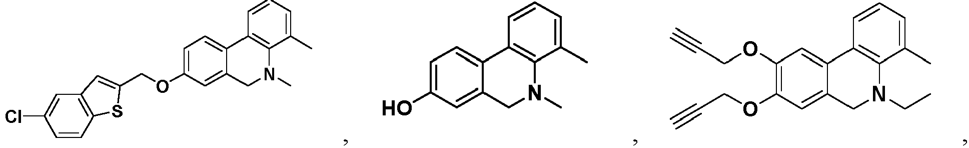 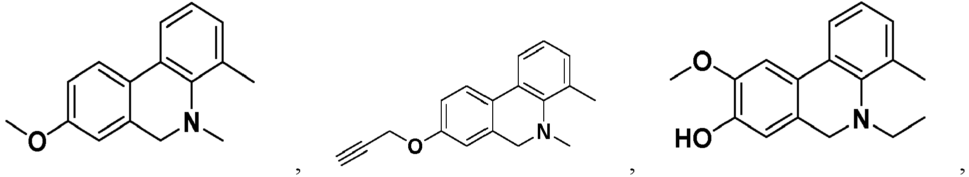 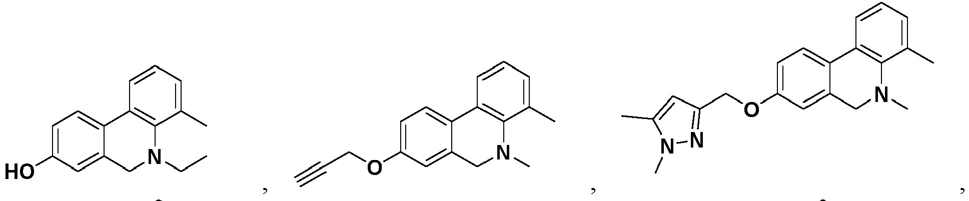 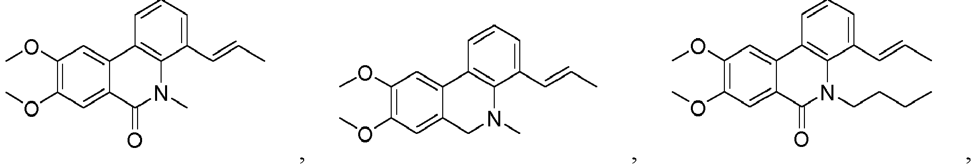 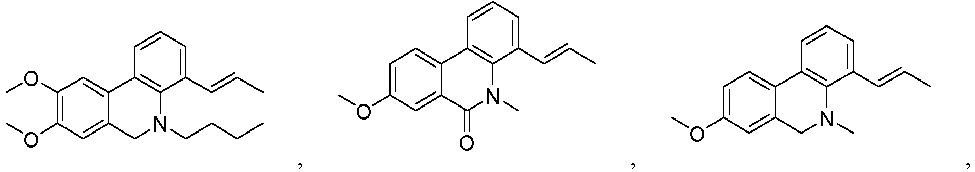 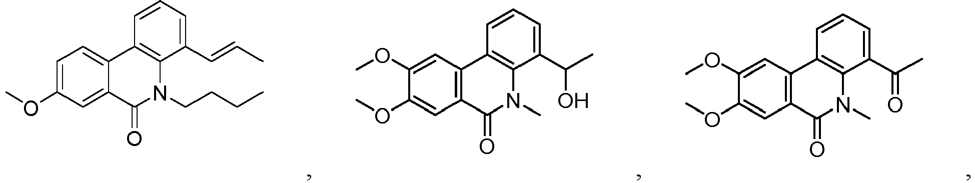 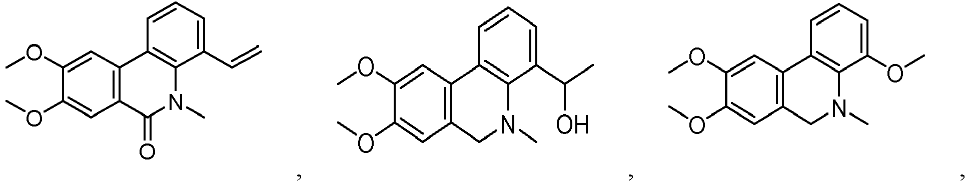 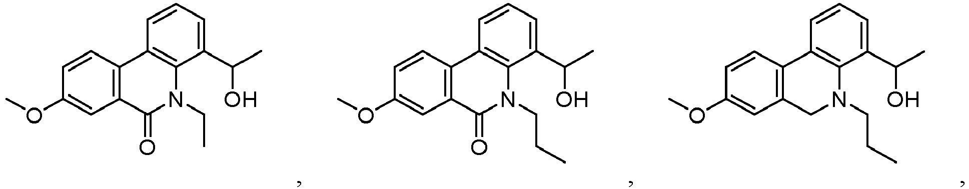 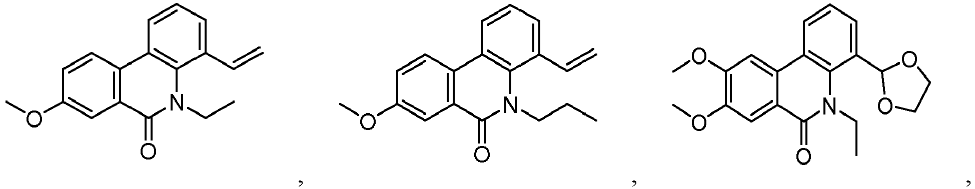 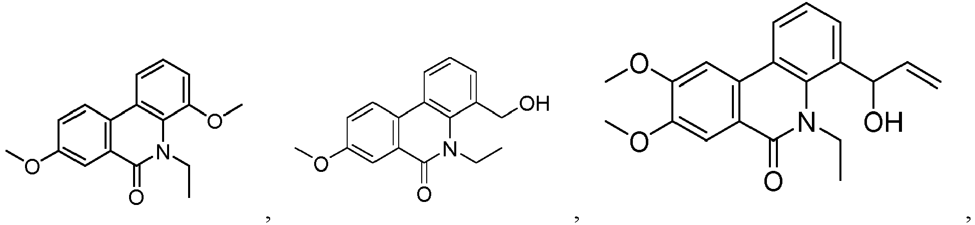 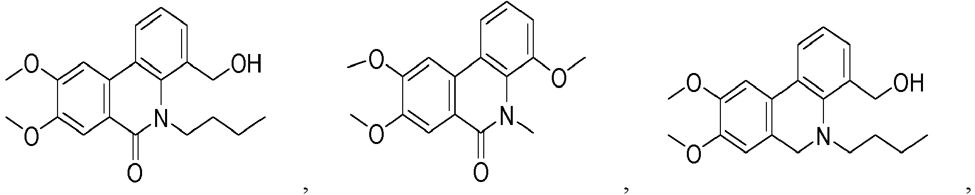 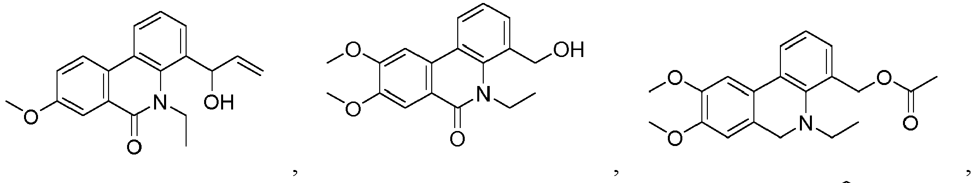 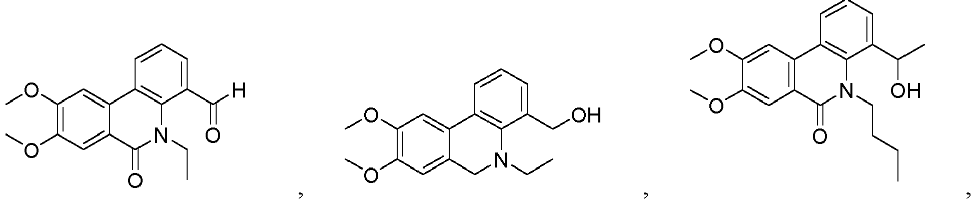 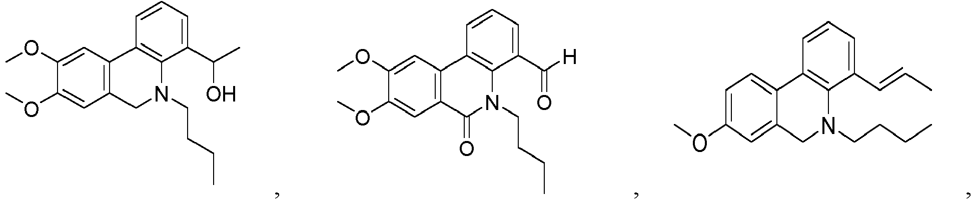 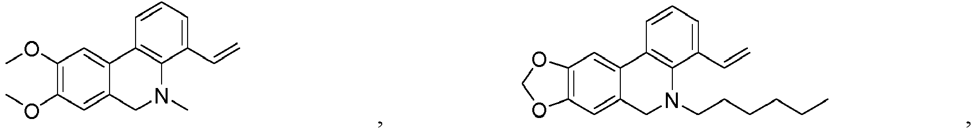 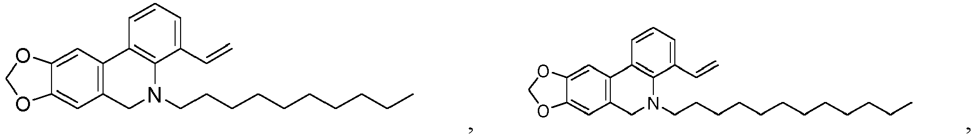 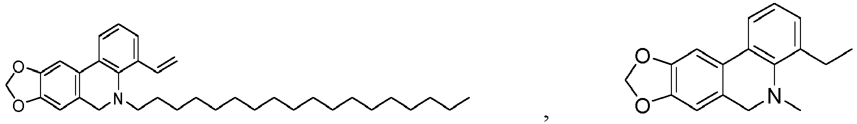 and

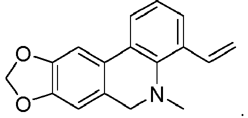

7. Use of a compound of formula I, or a salt thereof, or an optical isomer thereof, or a racemate thereof, or a solvate thereof, or a precursor thereof for preparing a composition or preparation, the composition or preparation is used to treat or prevent obesity or a related disease thereof, wherein the compound of formula I is as described in claim 1.

8. A pharmaceutical composition, comprising:
(a) The compound of formula I , or a salt thereof, or an optical isomer thereof, or a racemate thereof, or a solvate thereof, or a precursor thereof of claim 1; and
(b) a pharmaceutically acceptable carrier.

9. A kit containing:
(i) a first container, and an active ingredient (a1) a compound of formula I, or a salt thereof, or an optical isomer thereof, or a racemate thereof, or a solvate thereof, or a precursor thereof or a drug containing the active ingredient (a1) contained in the first container; wherein the compound of formula I, or a salt thereof, or an optical isomer thereof, or a racemate thereof, or a solvate thereof, or a precursor thereof is as defined in claim 1.

10. A method for preparing a pharmaceutical composition for treating or preventing the obesity or a related disease thereof, comprising the steps of: mixing the compound of formula I, or a salt thereof, or an optical isomer thereof, or a racemate thereof, or a solvate thereof or a precursor thereof of claim 1, with a pharmaceutically acceptable carrier, thereby forming a pharmaceutical composition.
